(19) Europäisches Patentamt
European Patent Office
Office européen des brevets

(11) Publication number: **0 400 861 A1**

(12) **EUROPEAN PATENT APPLICATION**

(21) Application number: **90305531.7**

(22) Date of filing: **22.05.90**

(51) Int. Cl.[5]: **C07D 319/06, C09K 19/30**

(30) Priority: **31.05.89 JP 138738/89**
**06.06.89 JP 143844/89**
**20.06.89 JP 157127/89**
**20.06.89 JP 157128/89**
**08.09.89 JP 233324/89**
**12.10.89 JP 265661/89**
**20.11.89 JP 301450/89**

(43) Date of publication of application:
**05.12.90 Bulletin 90/49**

(84) Designated Contracting States:
**CH DE GB LI**

(71) Applicant: **SEIKO EPSON CORPORATION**
**4-1, Nishishinjuku 2-chome**
**Shinjuku-ku Tokyo(JP)**

(72) Inventor: **Obikawa, Tsuyoshi**
**c/o Seiko Epson Corporation**
**3-5 Owa 3-chome, Suwa-shi, Nagano-ken(JP)**
Inventor: **Ikukawa, Shuji**
**c/o Seiko Epson Corporation**
**3-5 Owa 3-chome, Suwa-shi, Nagano-ken(JP)**
Inventor: **Nakayama, Jitsuko**
**c/o Seiko Epson Corporation**
**3-5 Owa 3-chome, Suwa-shi, Nagano-ken(JP)**

(74) Representative: **Caro, William Egerton et al**
**J. MILLER & CO. Lincoln House 296-302 High Holborn**
**London WC1V 7JH(GB)**

(54) **1,3-dioxane derivative and liquid crystal composition incorporating the same.**

(57) A 1,3-dioxane derivative of the general formula:

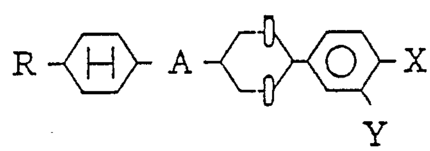

wherein, R is a straight chain alkyl group of 1 to 10 carbon atoms, A is a single bond or a $-CH_2-CH_2-$ group, X is F or CN, Y is either F when X is CN or H or F when X is F, and the cyclohexane ring and the 1,3-dioxane ring are each in the trans configuration.

EP 0 400 861 A1

## 1,3-DIOXANE DERIVATIVE AND LIQUID CRYSTAL COMPOSITION INCORPORATING THE SAME

This invention relates to 1,3-dioxane derivatives useful as components of nematic liquid crystal compositions used in liquid crystal devices and to liquid crystal compositions incorporating the same.

Liquid crystal display devices utilizing the electro-optic effect of a nematic liquid crystal composition have been applied in a wide range of fields. As modes of display, the dynamic scattering type, the guest host type, the twisted nematic (TN) type, the super twisted nematic (STN) type, the super twisted birefringence (SBE) type, etc. are known. As methods of driving these display devices, the static drive method, the time shared drive method (dynamic drive method), the active matrix drive method, the two frequency drive method, etc. have found acceptance.

The liquid crystal display devices, as compared with emission type display devices such as LED's, EL's and CRT's, possess the following prominent characteristics:

(1) They permit reduction in size and thickness.

(2) They require only low driving voltage.

(3) They have very small power consumption.

(4) They show high affinity for LSI's and operate with a simple drive circuit.

(5) Since they operate with incident light, their displays are clearly visible even under direct sunlight and do not readily tire a viewer's eyes even after long periods of use.

As a result of these characteristics, liquid crystal display devices particularly of the TN type have found extensive utility in watches, desk top electronic calculators, audio devices, games, instrument panels in automobiles, cameras, telephones and various measuring instruments. The range of fields to which they are applied is expected to expand further in the future. Efforts have been made to produce liquid crystal display devices with an increased display capacity. It has been demonstrated that the maximum number of scanning lines attainable in a liquid crystal display devices of the TN type using the time shared drive method is about 200. Liquid crystal display devices of the STN type and the SBE type have been developed with more scanning lines to the extent of verging on practical utility, with the allowable numbers of scanning lines approximating those of CRT's. Further, liquid crystal display devices of the TN type operated by the active matrix drive method allow a considerable addition to the number of scanning lines and have approached the stage of practical utility. The liquid crystal display devices of the STN type, amongst others, have been finding utility in display devices for personal computers and word processors. Liquid crystal display devices of the TN type operated by the active matrix drive method have been finding utility in liquid crystal colour televisions and are attracting attention as display devices promising to take the place of CRT's in the future.

Of the many characteristics which liquid crystal materials used for these liquid crystal devices are required to possess, those which are basic and are believed to be indispensable for all of the liquid crystal display devices are as follows:-

(1) The liquid crystal materials should suffer no colouration and exhibit stability thermally, optically, electrically and chemically.

(2) The liquid crystal materials should possess as wide nematic liquid crystal temperature ranges (MR) as possible in the neighbourhood of normal room temperature.

(3) The liquid crystal materials should show low threshold voltages ($V_{th}$) in their voltage luminance characteristics ($V-I_o$ characteristics) and their threshold voltages should possess only small temperature dependency.

(4) The $V-I_o$ characteristics of the liquid crystal materials should exhibit as high precipitousness ($\gamma$) as possible.

(5) The $V-I_o$ characteristics of the liquid crystal materials should exhibit as low dependency ($\alpha$) on viewers' visual angle as possible.

(6) The liquid crystal materials should possess high electro-optical response speeds.

Numerous liquid crystal compounds have been known to satisfy (1) amongst others mentioned above. However, no existing liquid crystal compound is known by itself to satisfy the second and subsequent characteristics mentioned above. In the present state of affairs, therefore, to fulfil of all of these characteristics there is no alternative but to use liquid crystal compositions prepared by mixing a plurality of nematic liquid crystal compounds or compounds similar thereto. As components of such liquid crystal compositions, while it is practically convenient to mix liquid crystal compounds of one and the same series, the characteristics aimed at cannot be attained unless liquid crystal compounds of different series are mixed. The liquid crystal compositions which are used in the liquid crystal display devices of the TN type or the STN type are of the Np type. Since liquid crystal compositions produced solely of the components of the

Np type possess inferior characteristics, liquid crystal compositions with more desirable characteristics are obtained by mixing components of the Np type together with components of the Nn type. The number of components making up such liquid crystal compositions generally falls in the neighbourhood of 7 to 8 but, at times, exceeds 20.

Now, the second and subsequent characteristics mentioned above will be described. The nematic liquid crystal temperature range (MR) mentioned with respect to (2) is required for practical purpose to extend between -20° to +60° C. If a liquid crystal display device is used outdoors, such as on instrument panels of automobiles, it is required to have a temperature range between -40° to +80° C. A desire to lower the lower limit ($T_L$) of the MR is satisfied by mixing liquid crystal compounds capable of forming a eutectic composition. The eutectic composition can be found in accordance with the following formula:

$$\ell n X_K = \frac{\Delta H_K}{R} \left( \frac{1}{T_K} - \frac{1}{T_L} \right)$$

$$\Sigma X_K = 1$$

In the formula, $X_K$ stands for the mole fraction of a component K at $T_L$, TK stands for the melting point of component K, $\Delta H_K$ stands for the heat of solution, and R stands for the gas constant. The values given by the formula are relatively close to those found with respect to components of one and the same series. However, more often than not, these values are totally inapplicable to components of different species. Thus, the know how of persons engaged in designing liquid crystal compositions has to be relied on in great measure. The upper limit ($T_U$) of the MR is determined by the following formula:

$T_U = \Sigma X_K T_{N\text{-}I,\,K}$

In this formula, $X_K$ stands for the mole fraction of component K and $T_N$-I,K stands for the material-isotronic liquid phase transition temperature (N-I point) of component K. Generally the value of $T_U$ determined by this formula is liable to be greater than the value actually found. The components of a liquid crystal composition are classified by phase transition point into three kinds, i.e. low temperature liquid crystal compounds, intermediate temperature liquid crystal compounds, and high temperature liquid crystal compounds. The low temperature liquid crystal compounds have their crystal nematic phase transition temperature (C-N point) or smectic nematic phase transition temperature (S-N point) below 50° C and their N-I points in the neighbourhood of 50° to 70° C and constitute the principal components of liquid crystal compositions and determine $T_L$. Examples of such low temperature liquid crystal compounds include those of the following series.

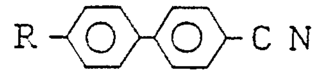
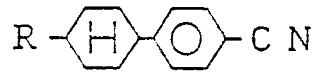
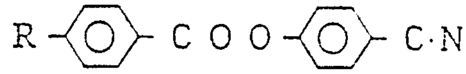
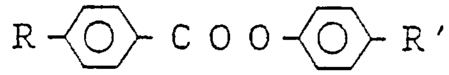
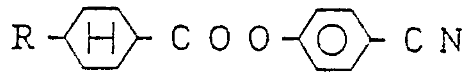
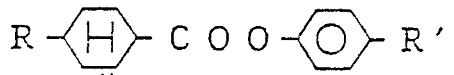
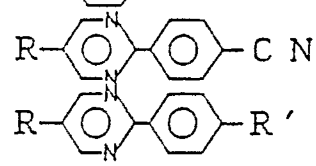

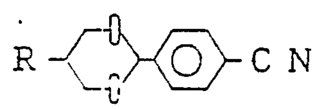

(wherein R and R′ independently stand for a straight chain alkyl group or an alkoxy group). The intermediate temperature liquid crystal compounds have their C-N point in the neighbourhood of 50° to 100° C and their N-I point approximately in the range of 100° to 200° C and may possibly constitute principal components of liquid crystal compositions. Examples of the compounds of this type are:

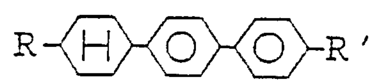
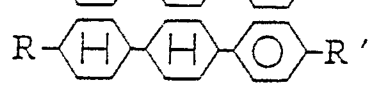
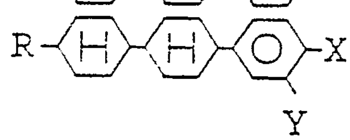

(wherein R and R′ independently stand for a straight chain alkyl group or an alkoxy group and X and Y independently stand for H or F). The high temperature liquid crystal compounds have their C-N point in the neighbourhood of 80° to 150° C and their N-I point above 200° C and are highly effective in raising $T_U$ values of liquid crystal compositions. They, however, have the disadvantage that they have large molecular weights and exhibit high degrees of viscosity. Since they generally do not manifest very satisfactory compatibility, they are not incorporated in a liquid crystal composition in a large amount. However, compounds which possess a smectic A phase below a nematic phase may be added in a relatively greater amount. Examples of such high temperature liquid crystal compounds are as follows:

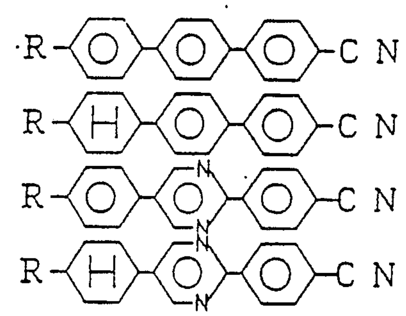

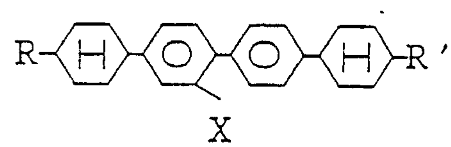

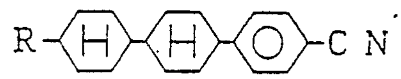

(wherein R and R′ independently stand for a straight chain alkyl group or an alkoxy group and X stands for H or F). In practice in production of a liquid crystal composition, liquid crystal compounds of all three kinds are suitably combined to meet the characteristics which the liquid crystal composition is required to possess.

The threshold voltage ($V_{th}$) or the V-$I_o$ characteristic in the case of the liquid crystal display device of the TN type, is expressed by the following formula:

$$V_{th} = \frac{\pi}{d}\sqrt{\frac{K_{11} + (K_{33} - 2K_{22})/4}{\varepsilon_0 \Delta\varepsilon}}$$

(wherein d stands for the thickness of a liquid crystal layer, $K_{11}$, $K_{22}$ and $K_{33}$ stand respectively for the elastic constants of spray, twist and bend, and $\epsilon_o$ stands for the vacuum dielectric constant and $\Delta\epsilon$ stands for the dielectric anisotropy). For the value of $V_{th}$ to be lowered, it suffices to add a liquid crystal compound which has a small elastic constant and a large positive dielectric anisotropy $\Delta\epsilon$. Examples of the liquid crystal compound which answer this description are as follows:

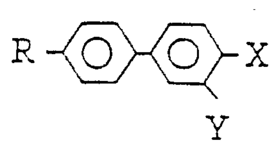

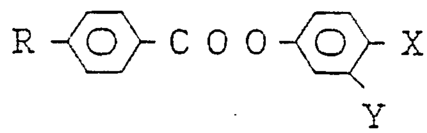

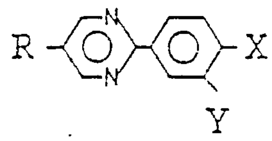

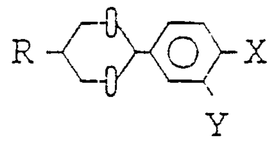

(wherein R stands for a straight chain alkyl group, X for F or CN, and Y for H or F). Generally $V_{th}$ tends to decrease in proportion as the temperature increases. This decrease is precipitous particularly in the neighbourhood of the N-I point. This trend is because of the dependency of the elastic constant on temperature. The addition of a liquid crystal compound of the Nn type is effective in counteracting the dependency of $V_{th}$ upon temperature. The addition of a liquid crystal compound of the Nn type results in lowering the dependency on temperature of the elastic constant of the liquid crystal composition because the dependence on temperature of the elastic constant of the liquid crystal compounds of the Nn type is smaller than that of the Np type. Though the same effect is obtained by the addition of a high temperature liquid crystal compound, this is accompanied by the disadvantage that $V_{th}$ is increased.

The precipitousness ($\gamma$) of the V-$I_o$ characteristic of (4) is expressed by the following formula, using the threshold voltage ($V_{th}$) and the saturated voltage ($V_{sat}$).

$$\gamma = \frac{V_{sat}}{V_{th}}$$

Generally, $V_{th}$ represents the voltage where the luminance is 10% and $V_{sat}$ the voltage where the luminance is 90%. Though $\gamma = 1$ is ideal, the value of $\gamma$ in practice is approximately in the range of 1.3 to 1.5. It is difficult to develop a liquid crystal compound possessing a smaller value of $\gamma$ than this. It has been demonstrated that in the case of a liquid crystal display device of the TN type, the value of $\gamma$ decreases in proportion as the value of $K_{33}/K_{11}$ decreases. The liquid crystal compounds possessing a pyrimidine skeleton have been known as liquid crystal compounds having small values of $\gamma$.

The dependency on visual angle of the V-$I_o$ characteristic (5), in the case of a liquid crystal display device of the TN type, originates in the pre-tilt which occurs between the plane which has under-gone orientation treatment and the liquid crystal material. This pre-tilt is a drawback peculiar to the TN type. It can be mitigated by decreasing the thickness of the liquid crystal layer or decreasing the value of birefringence ($\Delta n$) of the liquid crystal composition. Here, in the liquid crystal display device of the TN type, the transmittance T in the perfect absence of electric field is expressed by the following formula:

$T = \sin^2(\frac{\pi}{2}\sqrt{1 + u^2})/(1 + u^2)$

Here, u stands for $2d\,\Delta n/\lambda$. T = 0 results from the calculation based on this formula where u ≈ 2, 4, 6 ... It is known that the dependency on visual angle is smallest where u ≈ 2. Since it has been difficult to obtain a liquid crystal composition possessing a small value of $\Delta n$, the only recourse has consisted in decreasing the value of d. Since the existing production techniques permit no appreciable decrease in the value of d, the practice of producing a liquid crystal cell using such values of d and $\Delta n$ as to give d ≈ 4 has actually prevailed. The development of a liquid crystal compound possessing a small value of $\Delta n$, therefore, allows

realisation of a TN type cell having small dependency on visual angle sufficient to satisfy $\underline{u} \approx 2$.

As concerns the response speed (6), the initiating speed, $\tau_{ON}$, and the trailing speed $\tau_{OFF}$, in the case of a device of the TN type, are given by the following formulae:

$\tau_{ON} = \eta d^2/(\epsilon_0 \Delta\epsilon V^2 - \pi^2 K)$

$\tau_{OFF} = \eta d^2/K\pi^2$

In these formulae, $\eta$ stands for viscosity, V stands for applied voltage, and K stands for a value given by the following formula:

$K = K_{11} + (K_{33} - 2 K_{22})/4$

It is possible to attain a high response speed by decreasing the thickness of the layer of the liquid crystal composition, by lowering the viscosity of the liquid crystal composition, or by augmenting the value of K. From the standpoint of the liquid crystal composition, the enhancement of response speed is attained by using a compound with as small a value of $\eta$/K as possible. A compound which answers this description is generally known as a viscosity decreasing agent, examples being:

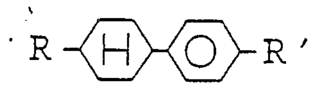

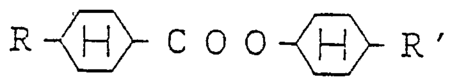

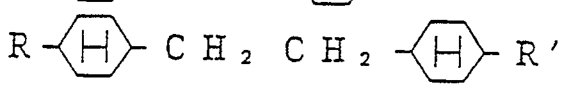

(wherein R and R′ independently stand for a straight chain alkyl or an alkoxy group).

Now, the relation between the time shared drive method of the devices of the TN type which are now used most widely and the characteristics (2) to (6) will be described. In the time shared drive method, the relation between the precipitousness ($\gamma$) and the largest allowable number of scanning lines (N) is expressed by the following formula:

$$N = \left(\frac{\gamma^2 + 1}{\gamma^2 - 1}\right)^2$$

From this formula, it is clearly noted that the value of N increases in proportion as the value of $\gamma$ approximates to 1. In practice, the voltage margin (M), defined by the following formula, is used as the criterion for the evaluation of the effectiveness of a given liquid crystal composition in the time shared drive method:

$$M = \frac{V_{th}(T=40℃, \phi=40°) - V_{sat}(T=0℃, \phi=10°)}{V_{th}(T=40℃, \phi=40°) + V_{sat}(T=0℃, \phi=10°)} \times 100$$

In this formula, $V_{th}$ and $V_{sat}$ stand for voltages at respective luminances of 10% and 50%, T stands for temperature, and $\phi$ stands for visual angle relative to the front side taken as 0°. This formula indicates that the following three requirements are important for realisation of the high time shared drive method.

(1) The precipitousness ($\gamma$) should be sharp.

(2) The extent of dependency of $V_{th}$ and $V_{sat}$ on temperature should be small.

(3) The dependency on visual angle ($\alpha$) should be small.

Heretofore, the following compounds have been known as liquid crystal compounds which improve these characteristics:

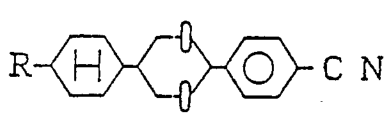

( I )

H-M. Vorbrodt et al. Mol. Cryst. Liq. Cryst. 123, 137 (1985).

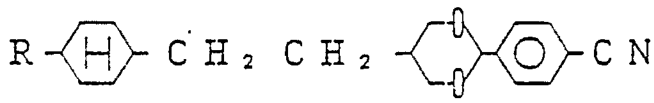 (II)

E. Kleinpeter et al. Tetrahedron, 44, 1809 (1988).

Though these nematic liquid crystal compounds possess high N-I points and small values of Δn and γ, they have the disadvantage that they do not exhibit very satisfactory compatibility with conventional liquid crystal compositions.

The present invention, therefore, seeks to provide a nematic liquid crystal compound having large Δε and small Δn and exhibiting highly satisfactory compatibility with other liquid crystal compounds. The present invention also seeks to provide a liquid crystal composition which includes a 1,3-dioxane derivative of the present invention admixed with other liquid crystal compounds and consequently enables high time shared drive at a low voltage.

According to the present invention there is provided a 1,3-dioxane derivative characterised by the general formula:

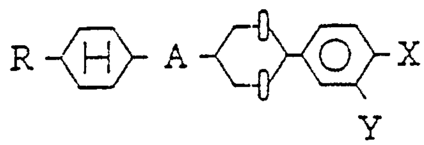

wherein, R is a straight chain alkyl group of 1 to 10 carbon atoms, A is a single bond or a $-CH_2-CH_2-$ group, X is F or CN, Y is either F when X is CN or H or F when X is F, and the cyclohexane ring and the 1,3-dioxane ring are each in the trans configuration.

The present invention also provides the following six 1,3-dioxane derivatives:

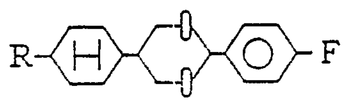 (1 - a)

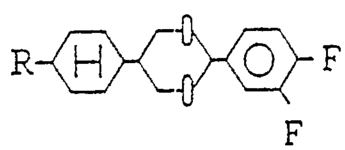 (1 - b)

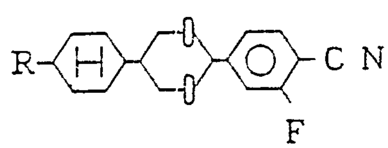 (1 - c)

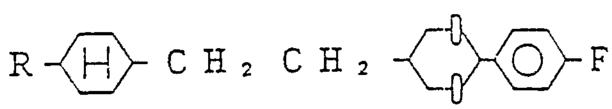 (1 - d)

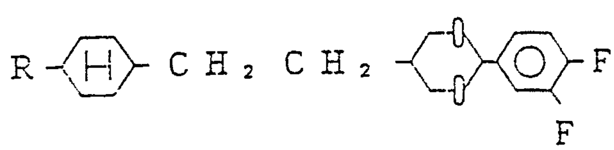 (1 - e)

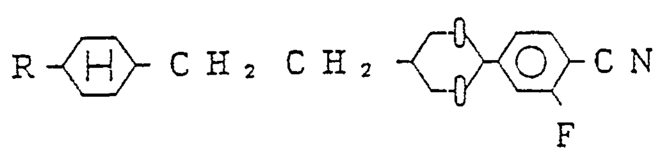 (1 - f)

wherein R stands for a straight chain alkyl group of 1 to 10 carbon atoms, the cyclohexane ring and the 1,3-dioxane ring being each in a trans configuration.

According to another aspect of the present invention, there is provided a liquid crystal composition containing at least one 1,3-dioxane derivative according to the present invention.

1,3-dioxane derivatives according to the present invention have the following characteristics.

(1) They possess relatively high nematic isotropic liquid phase transistion points (N-I points).

(2) Their dielectric anisotropy ($\Delta\epsilon$) is positive and large.

(3) Their birefringence ($\Delta\eta$) is small.

By mixing the compounds of the present invention with conventional liquid crystal compounds or compounds similar thereto, nematic liquid crystal compositions possessing the following properties can be obtained.

(1) They have wide nematic liquid crystal temperature ranges (MR).

(2) They have low threshold voltages ($V_{th}$) for voltage luminance characteristics ($V\text{-}I_o$ characteristics).

(3) They have wide visual angle ranges for the $V\text{-}I_o$ characteristics.

(4) They allow high time shared drive at a low voltage.

When the compounds of the present invention are admixed with the conventional liquid crystal compositions, the allowable mixing ratio is in the range of 1 to 50% by weight. In due consideration of such factors as compatibility, the mixing ratio is preferably in the range of 3 to 30% by weight. For this admixture, the 1,3-dioxane derivatives according to the present invention may be used singly. Where the admixture is intended for improvement of various characteristics, however, 1,3-dioxane derivatives according to the present invention are more effectively used jointly in the form of a combination of at least two members.

1,3-dioxane derivatives according to the present invention have resulted from the elimination of drawbacks of the following compounds:

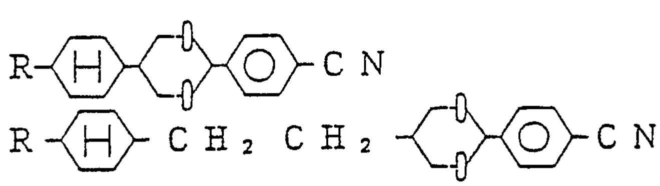
(I)
(II)

Of these compounds, those which contain $C_4H_9$ as the substituent R have the phase transition points shown in Table 1.

Table 1

| (In °C) | | | |
|---|---|---|---|
| | C • S • N • I | | |
| (1-a) | 70.0 | (77.7) | 145.0 |
| (1-b) | 89.8 | - | 109.7 |
| (1-c) | 89.6 | - | 175.1 |
| (1-d) | 95.6 | - | 119.4 |
| (1-e) | 83.0 | - | 87.0 |
| (1-f) | 63.5 | - | 142.8 |
| (I) | 74.6 | 91.5 | 221.2 |
| (II) | 72.2 | 91.7 | 179.4 |

It is clearly noted from this Table that the compounds (I) and (II) having CN substituted at the 4 position of the phenyl group have the highest N-I points, followed by the compounds (1-c) and (1-f) having CN substituted at the 4 position and F at the 3 position and the compounds (1-a) and (1-d) having F substituted at the 4 position, and compounds (1-b) and (1-e) having F substituted each at the 3 and 4 positions have the lowest N-I points and that the compounds having CN substituted at the 4 position have the highest C-N points, followed by the compounds having F substituted each at the 3 and 4 positions, the compounds having F substituted at the 4 position, and the compounds having F substituted at the 3 position, and the

compounds having F substituted at the 3 position and CN at the 4 position. When these compounds are classified by the method described above, the compounds (I) are rated as high temperature liquid crystal compounds and all of the other compounds as intermediate temperature liquid crystal compounds.

These compounds have been tested for compatibility with other liquid crystal compositions. The results of this test are shown in Table 4. From Table 4, it is noted that the 1,3-dioxane derivatives according to the present invention have improved compatibility over compounds (I) and (II). Since an intermediate temperature liquid crystal compound can be used as the main component for a liquid crystal composition when it possesses satisfactory compatibility, 1,3-dioxane derivatives according to the present invention mixed suitably with other liquid crystal compounds can be used as the main component of a resultant liquid crystal composition. The allowable ratio in which the compounds of this invention can be mixed with other liquid crystal compounds is considered to be approximately in the range of 1 to 50% by weight. Where the compatibility constitutes itself an important consideration, the mixing ratio is preferably in the range of 3 to 30% by weight. When 1,3-dioxane derivatives according to the present invention are with into other liquid crystal compounds in a ratio of less than 3% by weight, they do not conspicuously manifest their characteristics in the resultant liquid crystal composition. The other liquid crystal compounds with which 1,3-dioxane derivatives according to the present invention can be mixed are not particularly critical. 1,3-dioxane derivatives according to the present invention can be mixed with compounds of the following series or with compounds similar thereto:

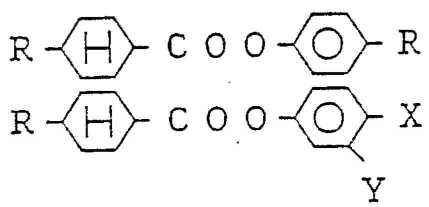

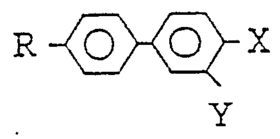

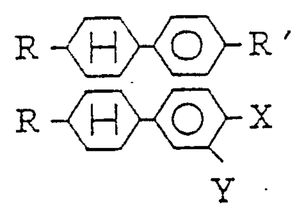

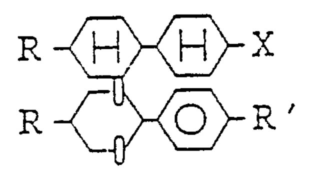

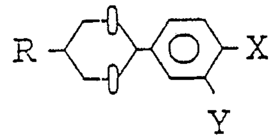

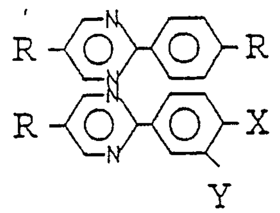

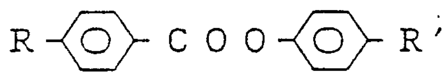

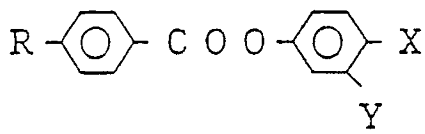

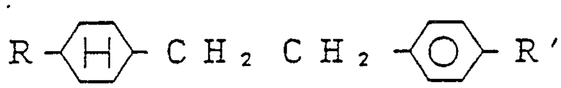

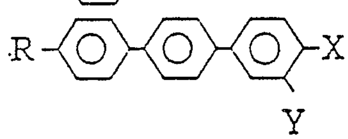

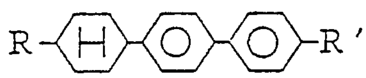

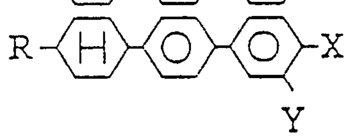

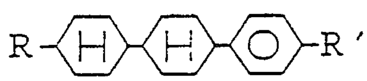

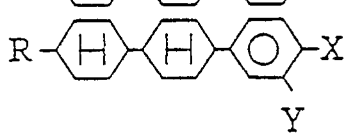

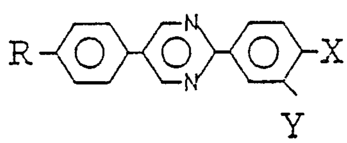

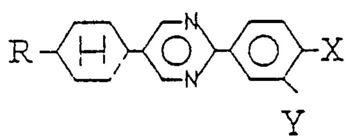

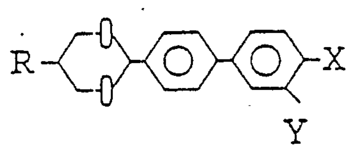

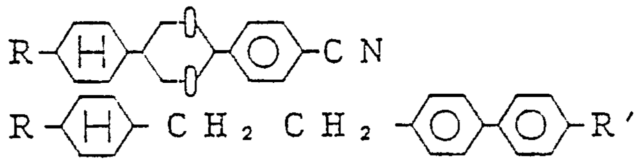

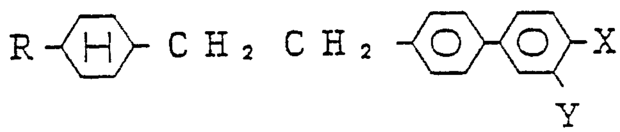

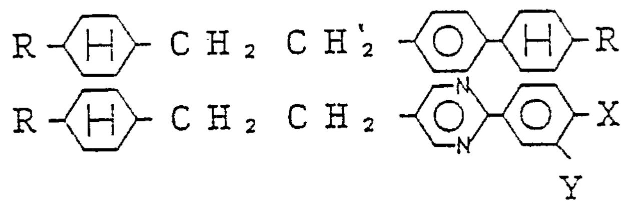

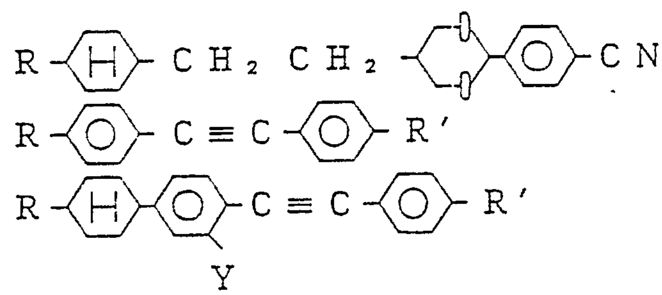

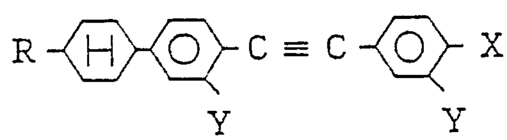

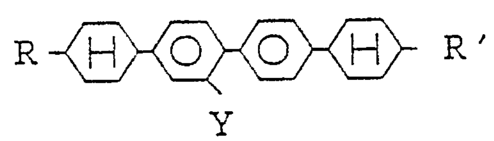

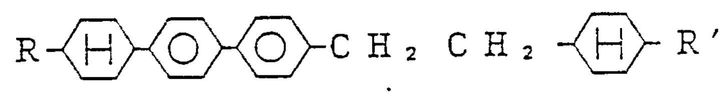

wherein R and R′ independently stand for a straight chain alkyl group, a alkoxy group, a straight chain alkenyl group, or a straight chain alkenyloxy group, X stands for CN or F, and Y for H or F.

The compounds enumerated above are only a very small proportion of the liquid crystal compounds available for inclusion in liquid crystal compositions according to the present invention.

The threshold voltages ($V_{th}$) and the magnitudes of temperature dependency, ΔT, of $V_{th}$ in a temperature range of 0° to 40°C which are found in admixtures of a commercially available liquid crystal composition with 10% by weight each of 1,3-dioxane derivatives according to the present invention, the compounds (I) and (II),

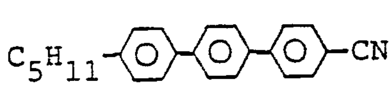 (III), and 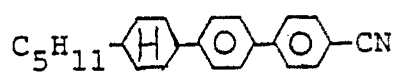 (IV)

are shown in Table 2 (R for $C_4H_9$).

Table 2

| Compound | (1-a) | (1-b) | (1-c) | (1-d) | (1-e) | (1-f) | (I) | (II) | (III) | (IV) |
|---|---|---|---|---|---|---|---|---|---|---|
| $V_{th}$ (V) | 2.51 | 2.42 | 2.19 | 2.50 | 2.38 | 2.23 | 2.38 | 2.45 | 2.51 | 2.47 |
| T(-V) | 0.28 | 0.30 | 0.31 | 0.25 | 0.31 | 2.32 | 0.31 | 0.31 | 0.29 | 0.29 |

It is noted from Table 4 that the compounds having F substituted at the 4 position equal the compounds (III), the compounds having F substituted each at the 3 and 4 positions equal the compounds having CN substituted at the 4 position, and the compounds having F substituted at the 3 position and CN at the 4 position have considerably lower magnitudes of $V_{th}$. Thus, the compounds (1-c) and (1-f) of the present invention are notably effective in lowering the magnitudes of $V_{th}$ of liquid crystal compositions and form the compounds indispensable to effecting high time shared drive at a low voltage. The compounds (1-b) and (1-e) of the present invention, though inferior to the compounds (I) and (II) in terms of the N-I point, exhibit an equal effect in lowering $V_{th}$, possess smaller magnitudes of $\Delta n$ than the compounds (I) and (II), and excel in compatibility. Thus, they are believed to find utility in a wider range than the compounds (I) and (II). The compounds (1-a) and (1-d) having F substituted at the 4 position have particularly small dependency of $V_{th}$ on temperature. The other compounds show this dependency substantially on the same level. Since the compounds (1-a) and (1-d) have relatively high N-I points and small magnitudes of $\Delta n$, they qualify as compounds indispensable to high time shared drive.

As concerns the precipitousness, the compounds of this invention register nagnitudes which are equal to those of the compounds (I) to (IV) and do not deserve to be called particularly prominent.

The magnitudes of $\Delta n$ found in the admixtures of a commercially available liquid crystal composition with 10% by weight each of 1,3-dioxane derivatives according to the present invention and the compounds (I) to (IV) are shown in Table 3.

Table 3

| Compound | (1-a) | (1-b) | (1-c) | (1-d) | (1-e) | (1-f) | (I) | (II) | (III) | (IV) |
|---|---|---|---|---|---|---|---|---|---|---|
| $\Delta n$ | 0.124 | 0.123 | 0.130 | 0.124 | 0.123 | 0.127 | 0.130 | 0.128 | 0.149 | 0.139 |

From Table 3 it is noted that the compounds having a large number of benzene rings and having CN substituted at the 4 position possess large magnitudes of $\Delta n$ and the compounds having F as a substituent possess small magnitudes of $\Delta n$. The use of the compounds (1-a), (1-b), (1-d) and (1-e) of the present invention, therefore, widens visual angle and realises high time shared drive. Because of their small magnitudes of $\Delta n$, these compounds permit production of liquid crystal cells satisfying $\underline{u} \approx 2$ without requiring the thickness of the cell to be appreciably decreased and also permit production of liquid crystal devices having a wide visual angle.

1,3-dioxane derivatives according to the present invention are not particularly excellent in viscosity and elastic constant over other tricyclic type liquid crystal compounds. They have a high response speed because the cell adapted to satisfy $\underline{u} \approx 2$ has a small cell thickness. Since the response speed is theoretically proportional inversely to the square (actually the 3/2 power) of the thickness of the cell, the decrease in the thickness of the cell is more effective than in improving the factor, $\eta/K$, of the liquid crystal composition.

As concerns the three requirements for the time share drive, the 1,3-dioxane derivatives according to the prevent invention have magnitudes of $\gamma$ equal to those of the other compounds. The compounds (1-a) and (1-d) have small magnitudes of dependency of $V_{th}$ on temperature. The compounds (1-a), (1-b), (1-d) and (1-e) have small magnitudes of $\alpha$. The use of the compounds (1-c) and (1-f) allows the drive voltage to be lowered. When compounds (1-a) to (1-f) are mixed singly with the conventional liquid crystal compositions, they produce liquid crystal compositions fit for high time shared drive. When mixtures of compounds (1-a) to (1-f) are mixed with conventional liquid crystal compositions, the resultant liquid crystal compositions exhibit still better performance. Particularly the use of compounds (1-c) or (1-f) in the mixture is conspicuously effective because it permits production of a liquid crystal composition capable of high time shared drive and allows a reduction in the pressure resistance of LSI.

In summary, the present invention embraces the six kinds of 1,3-dioxane derivatives which possess characteristics beneficial to high time shared drive. The liquid crystal compositions obtained by suitably incorporating 1,3-dioxane derivatives according to the present invention, therefore, are capable of high time shared drive at low voltage. These compounds possess highly satisfactory compatibility and are thoroughly miscible with all liquid crystal compounds. The mixing ratio of these compounds is preferably in the range of 3 to 30% by weight.

Now, the method for the production of 1,3-dioxane derivatives according to the present invention will be described below. A compound represented by the general formula:

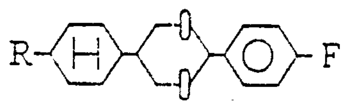

(1 - a) :

wherein R stands for a straight chain alkyl group and the cyclohexane ring and the 1,3-dioxane ring being each in the trans configuration, can be produced by a method indicated by Scheme 1 and Scheme 2 shown below, for example. Specifically in Scheme 1, from a 4-alkyl cyclohexanol (2) starting material, a 2-(4′-alkylcyclohexyl)propane-1,3-diol (5) is obtained by a three step reaction. In Scheme 2, a trans-2-(4′-fluorophenyl)-5-(trans-4′-alkylcyclohexyl)-1,3-dioxane, i.e. compound (1-a) of the present invention, is obtained by the reaction of compound (5) with a commercially available 4-fluorobenzaldehyde (6).

Now, the method for the production of compound (1-a) will be described in more detail.

Scheme 1

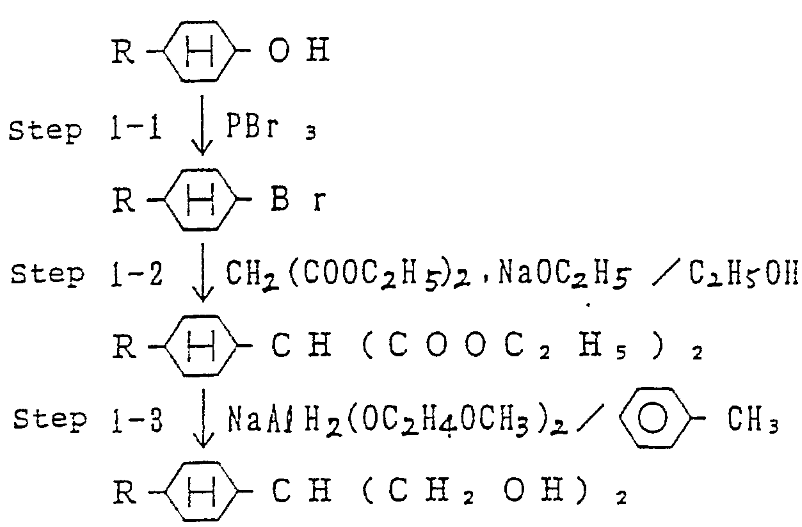


(2)

(3)

(4)

(5)

wherein R stands for a straight chain alkyl group of 1 to 10 carbon atoms and the cyclohexane ring is in the cis-trans mixed configuration.

Scheme 2

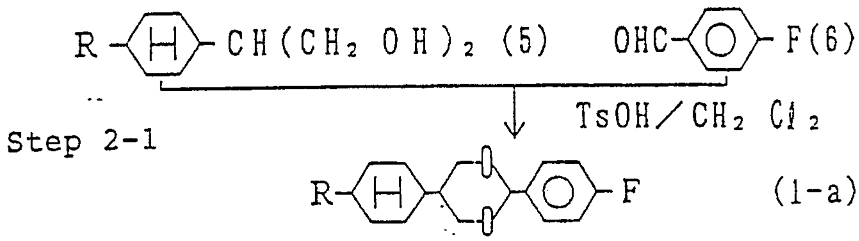


(1-a)

wherein R stands for a straight chain alkyl group of 1 to 10 carbon atoms and the cyclohexane ring and the 1,3-dioxane ring are each in the trans configuration.

Step 1-1: A 4-alkyl-1-bromocyclohexane (3) is obtained by brominating 4-alkylcyclohexanol (2) with phosphorus tribromide ($PBr_3$). Compound (2) used herein is a cis-trans mixture or a trans compound. When a $HBr$-$H_2SO_4$ system is used, the reaction gives a poor yield because the reaction produces 4-alkylcyclohexene in a large amount as a by-product.

Step 1-2: Diethyl 4-alkylcyclohexyl malonate (4) is obtained by causing compound (3) to react with diethyl malonate in anhydrous ethanol in the presence of sodium ethoxide ($NaOC_2H_5$). This reaction gives the product aimed at in a low yield because it forms 4-alkylcyclohexene.

Step 1-3: 2-(4′-alkylcyclohexyl)propane-1,3-diol (5) is obtained by reducing compound (4) in toluene by the use of sodium bis(methoxyethoxy) aluminium hydride ($NaAlH_2(OC_2H_4OCH_3)_2$).

Step 2-1: Trans-2-(4′-fluorophenyl)-5-(trans-4′-alkylcyclohexyl)-1,3-dioxane (1-a) is obtained by subjecting compounds (5) and 4-fluorobenzaldehyde to dehydration in dichloromethane in the presence of p-toluenesulphonic acid (TsOH) as a catalyst. In the product of this reaction, the cyclohexane ring and the 1,3-dioxane ring are both cis-trans mixtures. This product is re-crystallised for separation of the trans moiety.

Now, a compound represented by the general formula:

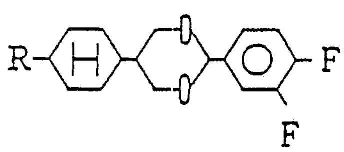

(1 - b)

wherein R stands for a straight chain alkyl group of 1 to 10 carbon atoms and the cyclohexane ring and the 1,3-dioxane ring are each in the trans configuration of the present invention can be produced by a method indicated by Scheme 1 and Scheme 3 shown below, for example.

Specifically, Scheme 1 is the same as the method for production of compound (1-a) and therefore further discussion is omitted here. In Scheme 3, a trans-2-(3′-4′-alkyl-cyclohexyl)-1,3-dioxane, i.e. compound (1-b) of the present invention is obtained by causing compound (5) to react with 3,4-difluorobenzaldehyde. Now, the method for the production of the 1,3-dioxane derivative (1-b) will be described in greater detail, but since Steps 1-1 to 1-3 are identical with those for the production of the compound (1-a), they are omitted here.

Scheme 3

Step 3-1: Trans-2-(3′,4′-difluorophenyl)-5-(trans-4′-alkylcyclohexyl)-1,3-dioxane (1-b) is obtained by causing compound (5) to react with a commercially available 3,4-difluorobenzaldehyde (7) in dichloromethane in the presence of the TsOH as a catalyst. The separation of the trans moiety is effected in the same manner as in Step 2-1.

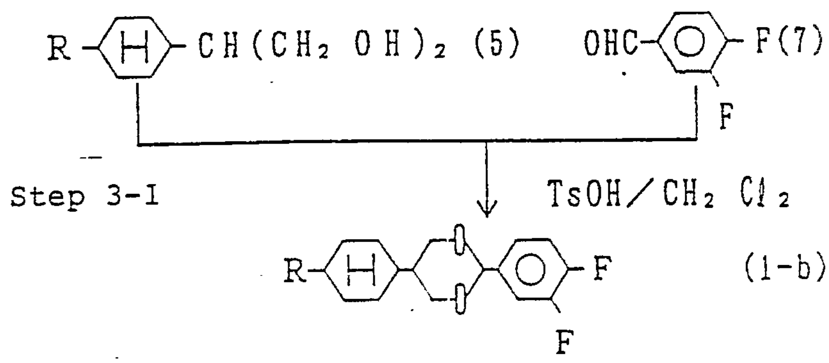


Now, a compound represented by the general formula:

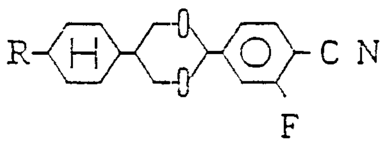

(1 - c)

wherein R stands for a straight chain alkyl group of 1 to 10 carbon atoms and the cyclohexane ring and the 1,3-dioxane ring are each in the trans configuration of the present invention is produced by the method indicated by Scheme 1, Scheme 4 and Scheme 5 shown below. Scheme 1 is identical with that for the production of compound (1-a) and therefore further discussion is omitted here. In Scheme 4, 4-bromo-3-fluorobenzaldehyde (11) is obtained by a three step reaction from a commercially available 4-bromo-2-fluoroaniline (8) as a starting material.

Scheme 4

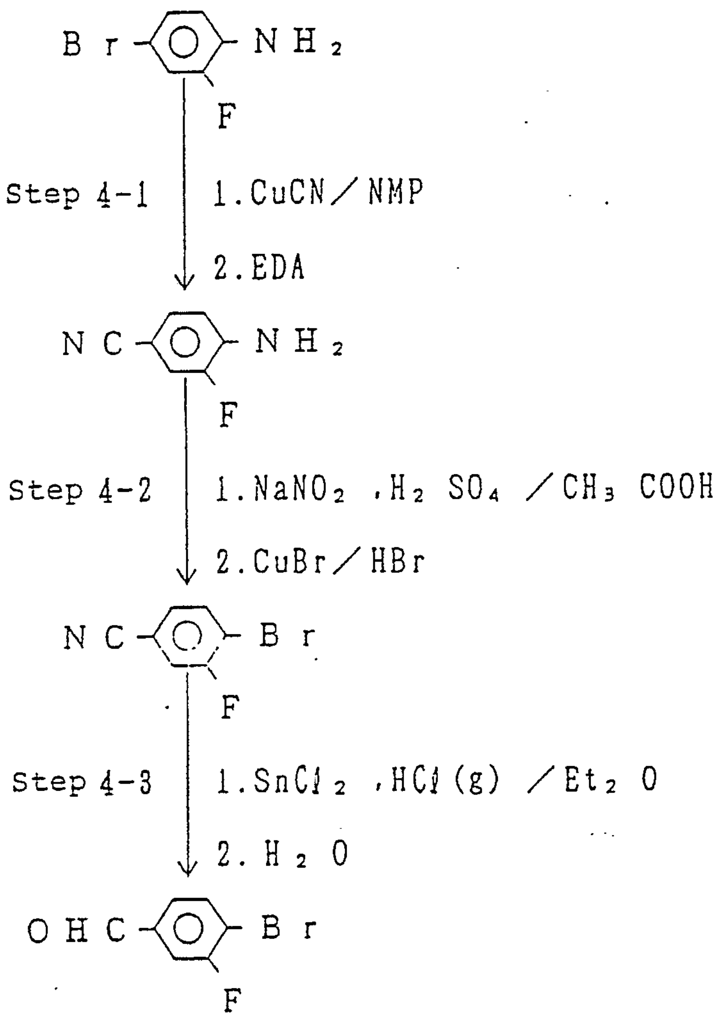


(8)

(9)

(10)

(11)

In Scheme 5, a trans-2-(4′-cyano-3′-fluorophenyl)-5-(trans-4′-alkylcyclohexyl)-1,3-dioxane, i.e. compound (1-c) of the present invention is obtained by a two step reaction from the compounds (5) and the compound (11).

Scheme 5

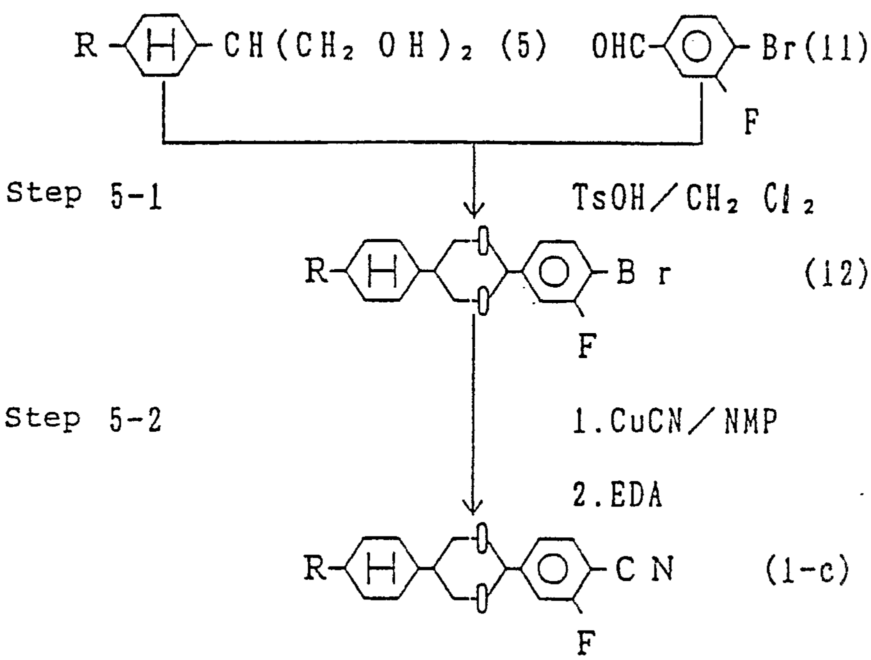


wherein R stands for a straight chain alkyl group of 1 to 10 carbon atoms.

Now, the method for the production of the 1,3-dioxane derivative (1-c) of the present invention will be described in detail.

Step 4-1: 4-amino-3-fluorobenzonitrile (9) is obtained by cyanating a commercially available 4-bromo-2-fluoroaniline (produced by Aldrich) in N-methyl-2-pyrrolidinone (NMP) by the use of copper (I) cyanide (CuCN) and decomposing the resultant copper complex with ethylene diamine (EDA). Optionally, a solution of iron (III) chloride in hydrochloric acid may be used for the decomposition of the copper complex.

Step 4-2: 4-bromo-3-fluorobenzonitrile (10) is obtained by diazodizing compound (9) in glacial acetic acid by the use of nitrocil hydrogen sulphate ($HSO_4$-$ONO_2$) prepared from sodium nitrite ($NaNO_2$) and concentrated sulphuric acid and brominating the resultant diazonium salt in hydrobromic acid by the use of copper (I) bromide (CuBr).

Step 4-3: 4-bromo-3-fluorobenzaldehyde (11) is obtained by treating compound (10) in diethyl ether by the use of tin (II) chloride ($SnCl_2$) saturated with hydrogen chloride gas thereby reducing the cyano group into an aldehyde group and then decomposing the resultant tin complex with hot water.

Step 5-1: A 2-(4′-bromo-3′-fluorophenyl)-5-(4-alkylcyclohexyl)-1,3-dioxane (12) is obtained by subjecting the compound (5) and the compound (11) to dehydration with dichloromethane in the presence of TsOH as a catalyst. In the compound (12), the cyclohexane ring and the 1,3-dioxane ring are both cis-trans mixtures.

Step 5-2: A trans-2-(4′-cyano-3′-fluorophenyl)-5-(trans-4′-alkylcyclohexyl)-1,3-dioxane (1-c) is obtained by cyanating the compound (12) in NMP by the use of CuCN, decomposing the resultant copper complex with EDA, and effecting selective separation of the trans moiety in the same manner as in Step 2-1. During the course of the cyanidation, part of the 1,3-dioxane ring is isomerised from the transfer to the cis configuration.

Now, a compound represented by the general formula:

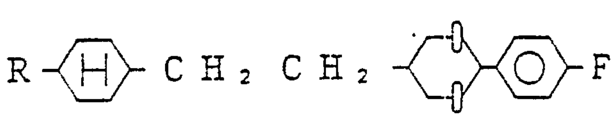
(1 - d)

wherein R stands for a straight chain alkyl group of 1 to 10 carbon atoms and the cyclohexane ring and the 1,3-dioxane ring are each in the trans configuration of the present invention can be produced by the method indicated in Scheme 6 and Scheme 7 shown below. Specifically, in Scheme 6, a 2-[2′-(trans-4′-alkylcyclohexyl)ethyl]propane-1,3-diol (21) is obtained by an eight step reaction using trans-4-alkylcyclohexane carboxylic acid (13) as a starting material. In Step 7, a trans-2-(4-fluorophenyl)-5-[2′-(trans-4′-alkyl-

cyclohexyl)ethyl]-1,3-dioxane, e.g. compound (1-d) of the present invention is obtained by the reaction of the compound (21) and the compound (6).

Scheme 6

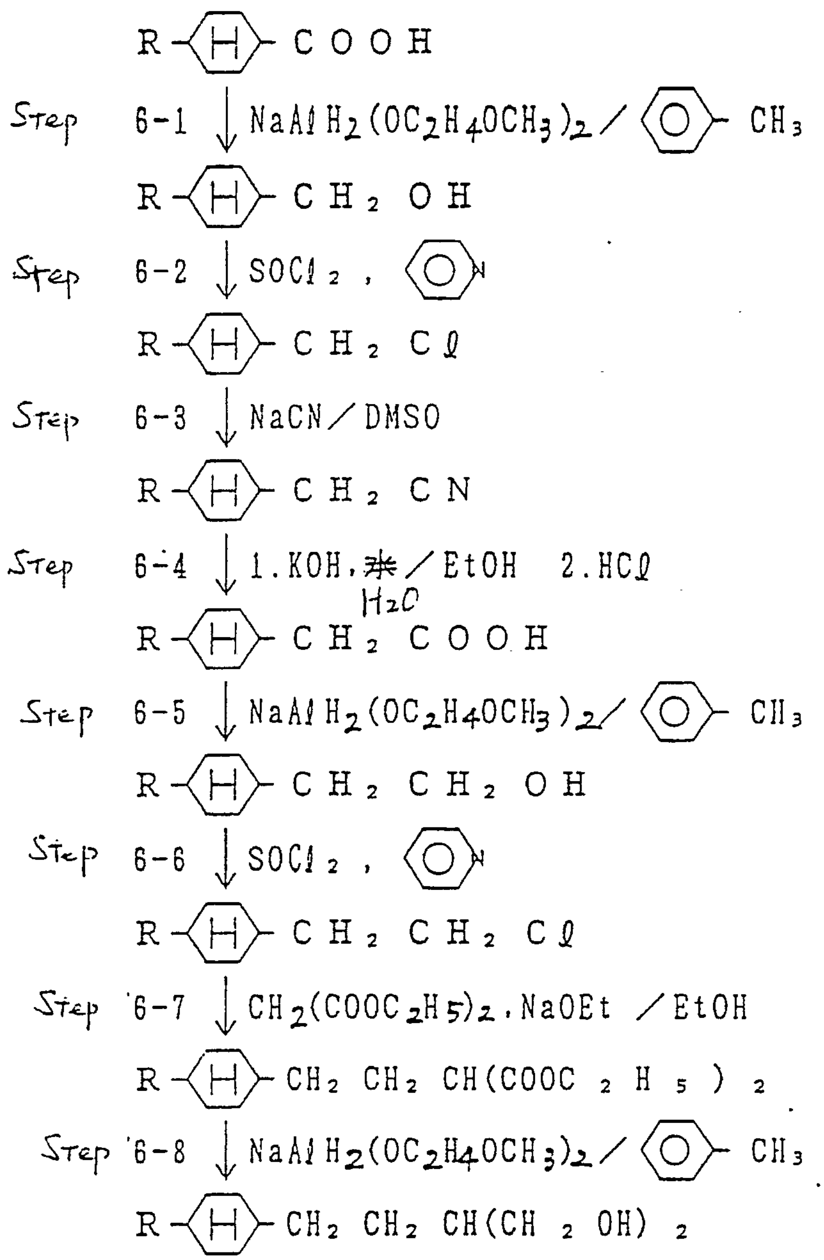


(13) (14) (15) (16) (17) (18) (19) (20) (21)

wherein R stands for a straight chain alkyl group of 1 to 10 carbon atoms and the cyclohexane ring is in the trans configuration.

Scheme 7

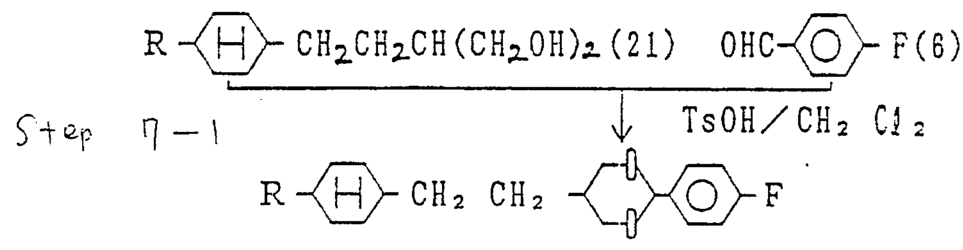


wherein R stands for a straight chain alkyl group of 1 to 10 carbon atoms and the cyclohexane ring and the 1,3-dioxane ring are each in the trans configuration.

Now, the method for the production of a 1,3-dioxane derivative (1-d) of the present invention will be desribed in detail.

Step 6-1: Trans-4-alkylcyclohexyl methanol (14) is obtained by reducing trans-4-alkylcyclohexane carboxylic acid (13) in toluene by the use of sodium bis(methoxyethoxy) aluminium hydride ($NaAlH_2(OC_2H_4OCH_3)_2$). Alternatively compound (4) may be obtained by converting compound (13) into a trans-4-alkylcyclohexane carbonyl chloride by the use of thionyl chloride ($SOCl_2$) and then reducing the product of conversion by the use of the same reducing agent.

Step 6-2: Trans-4-alkylcyclohexyl chloromethane (15) is obtained by chlorinating compound (14) in the presence of anhydrous pyridine by the use of $SOCl_2$.

Step 6-3: Trans-4-alkylcyclohexyl acetonitrile (16) is obtained by cyaniding compound (15) in dimethyl sulphoxide (DMSO) by the use of sodium cyanide (NaCN).

Step 6-4: Trans-4-alkylcyclohexyl acetic acid (17) is obtained by hydrolyzing compound (16) in ethanol by the use of potassium hydroxide (KOH) and neutralising the resultant hydrolyzate.

Step 6-5: 2-(trans-4′-alkylcyclohexyl)ethanol (18) is obtained by reducing compound (17) in toluene by the use of $NaAlH_2(OC_2H_4OCH_3)_2$. Alternatively, compound (18) may be obtained in high yield by chlorinating compound (17) into a trans-4-alkylcyclohexylacetyl chloride and reducing the product of this chlorination by following the procedure of Step 6-1.

Step 6-6: 2-(trans-4′-alkylcyclohexyl)-1-chloroethane (19) is obtained by chlorinating compound (18) by following the procedure of Step 6-2.

Step 6-7: Diethyl-2-(trans-4′-alkylcyclohexyl)-ethylmalonate (20) is obtained by an alkylating compound (19) and diethyl malonate in anhydrous ethanol by the use of sodium ethoxide (NaOEt). The product is obtained in a higher yield by using a 2-(trans-4′-alkylcyclohexyl)-1-bromoethane resulting from the bromination of compound (18) in the place of compound (19).

Step 6-8: 2-[2′-(trans-4′-alkylcyclohexyl)ethyl]-propane-1,3-diol (21) is obtained by reducing compound (20) in toluene by the use of $NaAlH_2(OC_2H_4OCH_3)_2$.

Step 7-1: Trans-2-(4′-fluorophenyl)-5-[2′-(trans-4′-alkylcyclohexyl)ethyl]-1,3-dioxane (1-d) is obtained by subjecting compound (21) and compound (6) to dehydration in dichloromethane by the use of TsOH as a catalyst and re-crystallising the product to dehydration thereby separating the trans moiety therefrom.

A compound represented by the general formula:

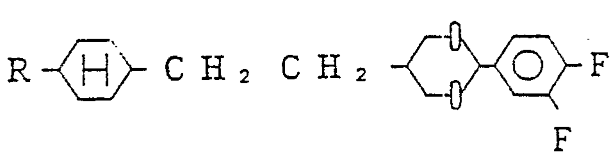 (1 - e)

wherein R stands for a straight chain alkyl group and the cyclohexane ring and the 1,3-dioxane ring are each in the trans configuration of the present invention can be produced by the method indicated by Scheme 6 and Scheme 8 shown below. Scheme 6 is the same as that which is involved in the production of compound (1-d) and therefore further discussion is omitted here. In Scheme 8, the trans-2-(3′,4′-difluorophenyl)-5-[2′-(trans-4″-alkyl-cyclohexyl)ethyl]-1,3-dioxane (1-e) of the present invention is obtained

by the reaction of compound (21) with compound (7).

Scheme 8

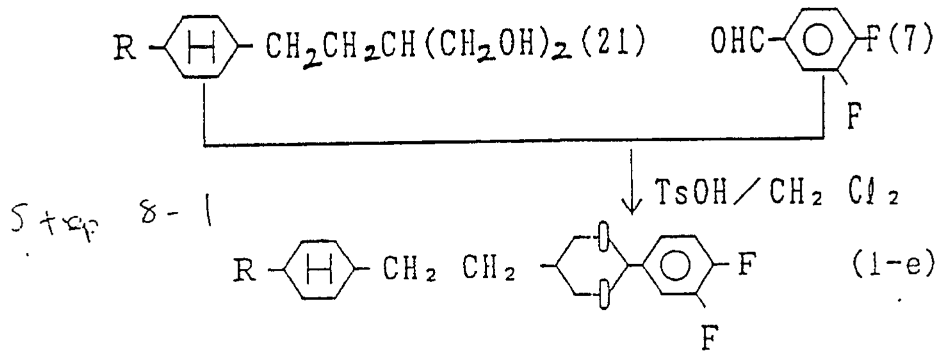


wherein R stands for a straight chain alkyl group of 1 to 10 carbon atoms and the cyclohexane ring and the 1,3-dioxane ring are each in the trans configuration.

Now, the method for the production of the 1,3-dioxane derivative (1-e) of the present invention will be described in detail.

Step 8-1: Trans-2-(3′,4′-difluorophenyl)-5-[2′-(trans-4″-alkyl cyclohexyl)ethyl]-1,3-dioxane (1-e) is obtained by subjecting compound (21) and compound (7) to dehydration in dichloromethane by the use of TsOH as a catalyst and re-crystallising the product of dehydration thereby separating the trans moiety from the product.

Finally, a compound represented by the general formula:

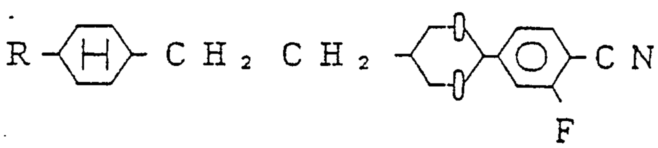
(1-f)

wherein R stands for a straight chain alkyl group and the cyclohexane ring and the 1,3-dioxane ring are each in the trans configuration of the present invention can be obtained by a method indicated by Scheme 6 and Scheme 9 shown below. Scheme 6 is the same as that involved in the production of the compound (1-d) and therefore further discussion is omitted here.

Scheme 9

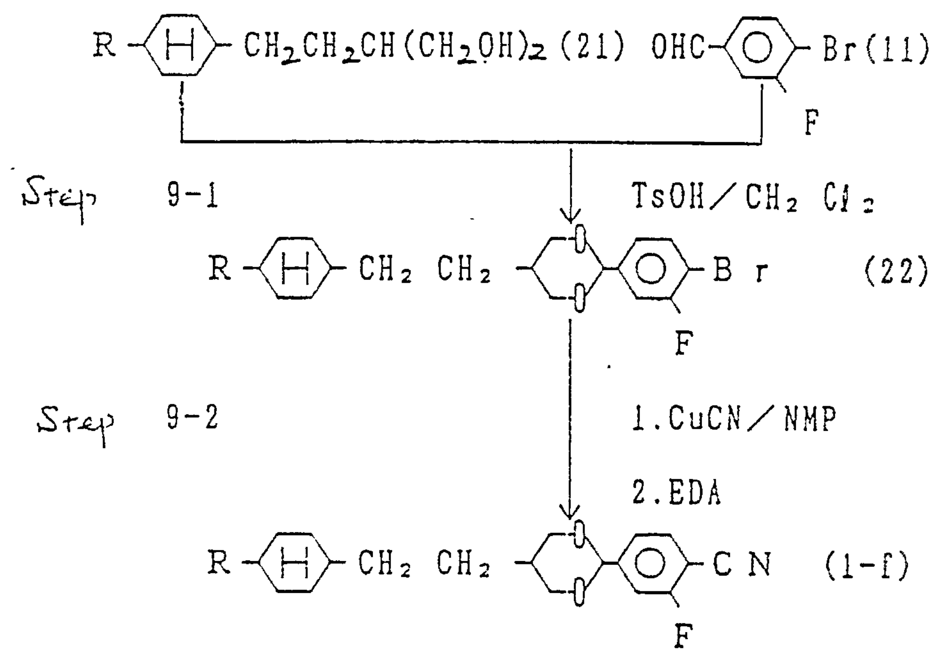


wherein R stands for a straight chain alkyl group of 1 to 10 carbon atoms and the cyclohexane ring and the 1,3-dioxane ring are each in the trans configuration.

In Scheme 9, a trans-2-(4′-cyano-3′-fluorophenyl)-5-[2′-(trans-4″-alkylcyclohexyl)ethyl]-1,3-dioxane (1-f) of the present invention is obtained by a two step reaction from compound (21) and compound (11).

Now, the method for the production of the 1,3-dioxane derivative (1-f) of the present invention will be described in detail.

Step 9-1: 2-(4′-bromo-3′-fluorophenyl)-5-[2′-(4″-alkylcyclohexyl)ethyl]-1,3-dioxane (22) is obtained by subjecting compound (21) and compound (11) to dehydration in dichloromethane by the use of TsOH as a catalyst.

Step 9-2: Trans-2-(4′-cyano-3′fluorophenyl)-5-[2′- (trans-4″-alkylcyclohexyl)ethyl]-1,3-dioxane (1-f) is obtained by cyaniding compound (22) in NMP by the use of CuCN, decomposing the resultant copper complex with EDA, and re-crystallising the product of decomposition thereby separating the trans moiety therefrom by following the procedure of Step 5-2.

Incidentally, the two compounds of the following formulae:

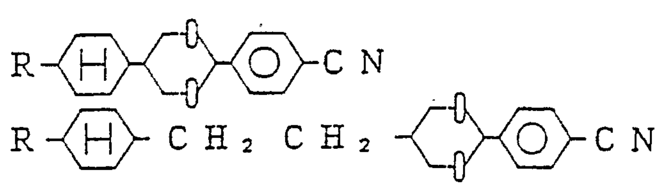

( I )

( II )

wherein R stands for a straight chain alkyl group and the cyclohexane ring and the 1,3-dioxane ring are each in the trans configuration can be produced by the following schemes respectively. First, the compound (I) is obtained by subjecting compound (5) and 4-cyanobenzaldehyde (23) to dehydration in dichloromethane by use of TsOH as a catalyst and re-crystallising the product of dehydration thereby separating the trans moiety therefrom as indicated in Scheme 10.

Scheme 10

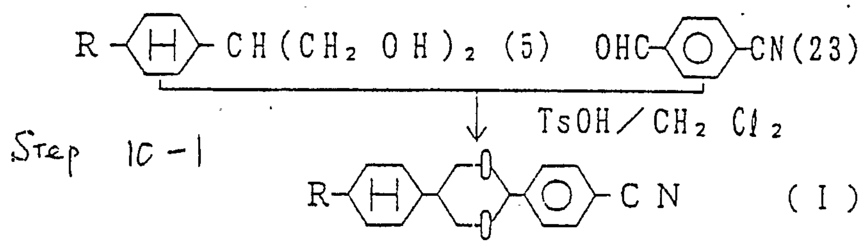


wherein R stands for a straight chain alkyl group and the cyclohexane ring and the 1,3-dioxane ring are each in the trans configuration.

Compound (II) is produced by subjecting compound (21) and compound (23) to dehydration in dichloromethane by the use of TsOH as a catalyst and re-crystallising the product of dehydration thereby separating the trans moiety therefrom as indicated in Scheme 11.

Scheme 11

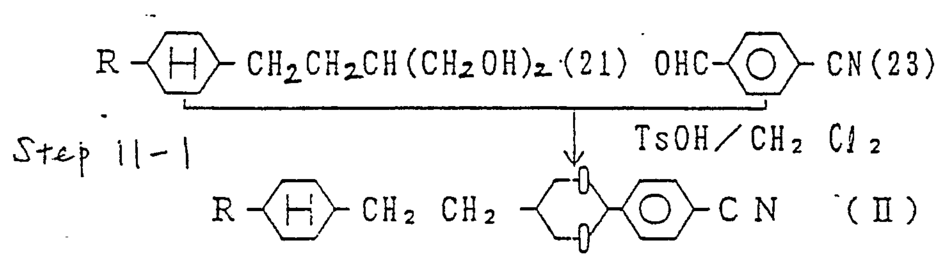


wherein R stands for a straight chain alkyl group and the cyclohexane ring and the 1,3-dioxane ring are each in the trans configuration.

This invention will be described more specifically below with reference to the following Examples.

Example 1. Method for production of 2-4′-propylcyclohexyl)propane-1,3-diol (Scheme 1).

Step 1-1: When 142 g (1.0 mol) of trans-4-propylcyclohexanol was kept stirred at room temperature and 136 g (0.5 mol) of $PBr_3$ was added dropwise thereto, they reacted exothermically and reached the temperature of about 80° C. The reaction product was stirred over a hot water bath at 70° C for one hour, then cooled to room temperature, and poured into iced water to effect decomposition of excess $PBr_3$. The oily layer was separated and the water layer was extracted with chloroform. They were re-combined and washed with water. The washed mixture was distilled to expel the chloroform. The residual oily substance was distilled under a vacuum (b.p. 97° C/14 mmHg), to obtain 180 g (0.88 mol) of 4-propyl-bromocyclohexane.

Step 1-2: In a solution of 31 g (1.35 mol) of sodium in 660 $cm^3$ of anhydrous ethanol, 216 g (1.35 mol) of diethyl malonate and 180 g (0.88 mole of 4-propyl-1-bromocyclohexane were sequentially dissolved in the order mentioned. Over a hot water bath, the resultant solution was refluxed for ten hours. The reaction product was cooled to room temperature. NaBr crystals which were consequently formed were separated by filtration and the filtrate was distilled to expel ethanol. The residue of the distillation was combined with water to effect separation of the oily layer. The water layer was extracted with chloroform, combined with the oily layer, and washed with water. The washed mixture was distilled to expel chloroform. By subjecting the residual oily substance to distillation under a vacuum (b.p. 138° C/4,4 mmHg), there was obtained 108 g (0.38 mol) of diethyl 4-propylcyclohexyl malonate. As an initial fraction, about 50 g of 4-propylcyclohexene was obtained.

Step 1-3: A solution obtained by dissolving 340 $cm^3$ (1.2 mol) of 70% $NaAlH_2(OC_2H_4OCH_3)_2$ solution in toluene in 340 $cm^3$ of anhydrous toluene was kept stirred at room temperature and 57 g (0.2 mol) of diethyl 4-propylcyclohexyl malonate was added dropwise thereto over a period of thirty minutes. The resultant solution was stirred over a hot water bath at 80° to 90° C for five hours and then cooled to room temperature. To the cooled solution, 50 $cm^3$ of water and 100 $cm^3$ of aqueous 15% hydrochloric acid solution were sequentially added dropwise thereto while in a continuously stirred state. The oily layer was separated and the water layer was extracted with toluene. The two layers were recombined and washed with an aqueous 10% hydrochloric acid solution and water. The washed mixture was distilled to expel toluene. The residue was recrystallised from hexane, to obtain 18 g (0.09 mol) of 2-(4-propylcyclohexyl)-propane-1,3-diol.

Example 2. Method for production of trans-2-(4′-fluorophenyl)-5-(trans-4′-propylcyclohexyl)-1,3-dioxane (Scheme 2).

Step 2-1: In 40 $cm^3$ of dichloromethane, 1.5 g (0.008 mol) of 2-(4-propylcyclohexyl)propane-1,3-diol, 1.4 g (0.01 mol) of 4-fluorobenzaldehyde (produced by Aldrich), and 0.1 g of TsOH were refluxed, over a hot water bath fitted with a Dean-Stark trap, for three hours with any water formed continuously removed from the reaction system. The reaction solution was washed with water and distilled to expel dichloromethane. The residue of the distillation was re-crystallised from a mixed solvent of acetone and methanol, to obtain 1.1 g (0.004 mol) of trans-2-(4′-fluorophenyl)-5-(trans-4′-propylcyclohexyl)-1,3-dioxane. When this compound was tested for phase transition point with DSC, the following results were obtained.

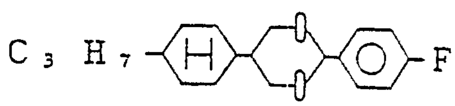

$$C \xrightarrow{82.4^\circ C} N \xrightarrow{146.2^\circ C} I$$

wherein C stands for crystal phase, N for nematic phase, and I for isotropic liquid phase.

The following compounds were produced by following the procedure of Example 2.

Trans-2-(4′-fluorophenyl)-5-(trans-4′-methylcyclohexyl)-1,3-dioxane;

Trans-2-(4′-fluorophenyl)-5-(trans-4′-ethylcyclohexyl)-1,3-dioxane;

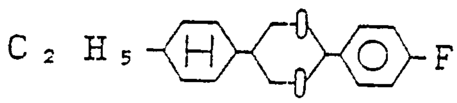

$$C \xrightarrow{81.3^\circ C} N \xrightarrow{142.0^\circ C} I$$

Trans-2-(4′-fluorophenyl)-5-(trans-4′-butylcyclohexyl)-1,3-dioxane;

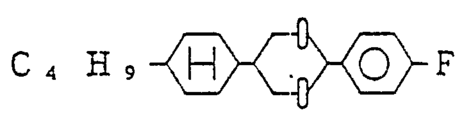

$$C \xrightarrow{70.0^\circ C} N \xrightarrow{145.0^\circ C} I$$
$$N \xrightarrow{77.7^\circ C} S$$

Trans-2-(4′-fluorophenyl)-5-(trans-4′-pentylcyclohexyl)-1,3-dioxane;

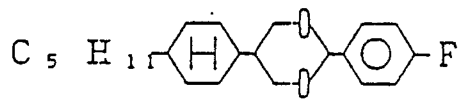

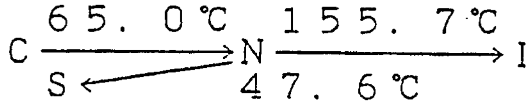


Trans-2-(4′-fluorophenyl)-5-(trans-4′-hexylcyclohexyl)-1,3-dioxane;

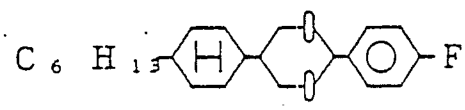

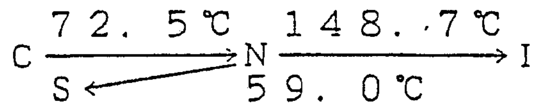


Trans-2-(4′-fluorophenyl)-5-(trans-4′-heptylcyclohexyl)-1,3-dioxane;
Trans-2-(4′-fluorophenyl)-5-(trans-4′-octylcyclohexyl)-1,3-dioxane;
Trans-2-(4′-fluorophenyl)-5-(trans-4′-nonylcyclohexyl)-1,3-dioxane;
Trans-2-(4′-fluorophenyl)-5-(trans-4′-decylcyclo hexyl)-1,3-dioxane.

Example 3. Method for production of trans-2-(3′,4′-difluorophenyl)-5-(trans-4′-propylcyclohexyl)-1,3-dioxane. (Scheme 3)

Step 3-1: In 40 $cm^3$ of dichloromethane, 1.5 g (0.008 mol) of 2-(4′-propylcyclohexyl)propane-1,3-diol, 1.5 g (0.01 mol) of 3,4-difluorobenzaldehyde (produced by Aldrich), and 0.1 g of TsOH were refluxed, over a hot water bath fitted with a Dean-Stark trap, for three hours, with any water formed continuously removed from the reaction system. The reaction solution was washed with water and distilled to expel dichloromethane. The residue of distillation was re-crystallised from a mixed solvent of acetone and methanol, to obtain 1.6 g (0.005 mol) of trans-2-(3′,4′-difluorophenyl)-5-(trans-4′-propylcyclohexyl)-1,3-dioxane.

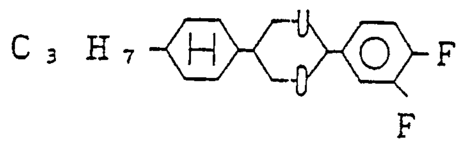

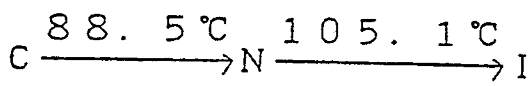


The following compounds were produced by following the procedure of Example 3.
Trans-2-(3′,4′-difluorophenyl)-5-(trans-4′-methylcyclohexyl)-1,3-dioxane;
Trans-2-(3′,4′-difluorophenyl)-5-(trans-4′-ethylcyclohexyl)-1,3-dioxane;

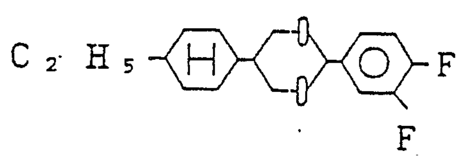

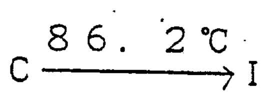

Trans-2-(3′,4′-difluorophenyl)-5-(trans-4′-butylcyclohexyl)-1,3-dioxane;

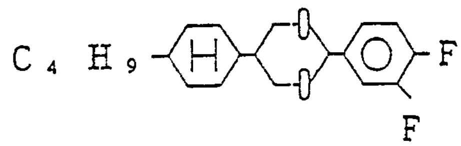

$$C \xrightarrow{89.8\,°C} N \xrightarrow{109.7\,°C} I$$

Trans-2-(3′,4′-difluorophenyl)-5-(trans-4′-pentylcyclohexyl)-1,3-dioxane;

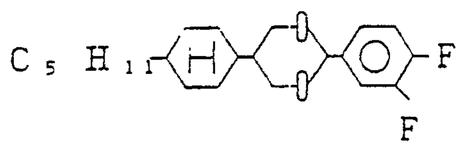

$$C \xrightarrow{77.0\,°C} N \xrightarrow{113.8\,°C} I$$

Trans-2-(3′,4′-difluorophenyl)-5-(trans-4′-hexylcyclohexyl)-1,3-dioxane;

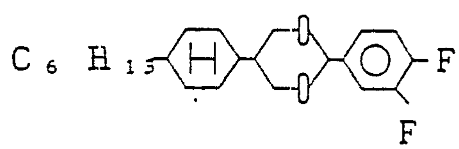

$$C \xrightarrow{70.1\,°C} N \xrightarrow{115.0\,°C} I$$

Trans-2-(3′,4′-difluorophenyl)-5-(trans-4′-heptylcyclohexyl)-1,3-dioxane;
Trans-2-(3′,4′-difluorophenyl)-5-(trans-4′-octylcyclohexyl)-1,3-dioxane;
Trans-2-(3′,4′-difluorophenyl)-5-(trans-4′-nonylcyclohexyl)-1,3-dioxane;
Trans-2-(3′,4′-difluorophenyl)-5-(trans-4′-decylcyclohexyl)-1,3-dioxane.

Example 4. Method for production of 4-bromo-3-fluorobenzaldehyde (Scheme 4).

Step 4-1: A solution obtained by dissolving 50 g (0.26 mol) of 4-bromo-2-fluoroaniline and 30 g (0.34 mol) of CuCN in 130 $cm^3$ of NMP was refluxed with a mantle heater for two hours. The reaction solution was cooled to room temperature. The cooled reaction product and 42 $cm^3$ of EDA added thereto were poured into 300 $cm^3$ of iced water. The resultant mixture was extracted with chloroform and washed with an aqueous EDA solution and water. The washed mixture was distilled to expel chloroform. The residue of the distillation was dissolved in ether washed with water to expel the engulfed NMP. The residue was distilled under a vacuum (b.p. 165° C/23 mmHg), to obtain 28 g (0.21 mol) of 4-amino-3-fluorobenzonitrile. The distillation was performed under an alleviated degree of vacuum because the product was so sublimable that the crystals adhered to the output of the distillation tube and clogged the tube.

Step 4-2: Over an iced water bath, 120 $cm^3$ of concentrated sulphuric acid was kept stirred and 15 g (0.22 mol) of $NaNO_2$, pulverised at a speed slow enough to keep the temperature below 30° C, was added thereto piecemeal. Over a hot water bath at 50° C, the resultant mixture was stirred until the crystals of $NaNO_2$ were thoroughly dissolved. The solution was kept stirred over an ice water bath and 200 $cm^3$ of

glacial acetic acid was added dropwise thereto over a period of thirty minutes. The resultant mixture was kept stirred and 28 g (0.21 mol) of 4-amino-3-fluorobenzonitrile was added thereto at a rate slow enough for the temperature to be kept in the range of 20° to 25° C. At this temperature, the resultant mixture was stirred until the crystals were thoroughly dissolved. Consequently, there was prepared a diazonium salt solution. A solution of 44 g (0.3 mol) of CuBr in 120 $cm^3$ of an aqueous 47% hydrobromic acid solution was vigorously stirred over an iced water bath and the diazonium salt solution prepared in advance as described above was added dropwise thereto over a period of one hour. After the dropwise addition was completed, the resultant mixture was stirred over an iced water bath for two hours and then left standing overnight. The crystals consequently formed were separated by filtration and washed in glacial acetic acid. The crystals were recrystallised from acetone, to obtain 31 g (0.16 mol) of 4-bromo-3-fluorobenzonitrile.

Step 4-3: A mixture consisting of 59 g (0.31 mol) of anhydrous $SnCl_2$ and 320 $cm^3$ of diethyl ether was placed over an iced water bath and, saturated until the $SnCl_2$ assumed a liquid state with hydrogen chloride gas which had been generated by dropwise addition of concentrated sulphuric acid to concentrated hydrochloric acid and then dried with concentrated sulphuric acid. The resultant mixture and 31 g (0.16 mol) of 4-bromo-3-fluorobenzonitrile added all at once thereto were stirred at room temperature for one hour and then left standing overnight. The reaction product and 300 $cm^3$ of water added thereto were stirred over a hot water bath at 50° C and then distilled to expel diethyl ether. The oily layer was separated and the water layer was extracted with ether. The two layers were combined, washed with water, and dried overnight with anhydrous $Na_2SO_4$. The resultant dried mixture was distilled to expel diethyl ether. The residue of the distillation was distilled to expel diethyl ether. The residue of the distillation was distilled under a vacuum (b.p. 122° C/25 mmHg), to obtain 26 g (0.13 mol) of 4-bromo-3-fluorobenzaldehyde.

Example 5. Method for production of trans-2-(4′-cyano-3′-fluorophenyl)-5-(trans-4′-propylcyclohexyl)-1,3-dioxane (Scheme 5).

Step 5-1: A solution of 4.0 g (0.02 mol) of 2-(4′-propylcyclohexyl)-propane-1,3-diol, 5.1 g (0.025 mol) of 4-bromo-3-fluorobenzaldehyde, and 0.2 g of TsOH in 100 $cm^3$ of dichloromethane was refluxed, over a hot water bath fitted with a Dean-Stark trap, for three hours, with any water formed continuously removed from the reaction system. The resultant reaction solution was washed with water and distilled to expel dichloromethane. By re-crystallising the residue from acetone, there was obtained 6.2 g (0.016 mol) of 2-(4′-bromo-3′-fluorophenyl)-5-(trans-4′-propylcyclohexyl)-1,3-dioxane. This compound contained about 10% of the cis form of a 1,3-dioxane ring.

Step 5-2 : In 40 $cm^3$ of NMP, 6.2 g (0.016 mol) of 2-(4′-bromo-3′-fluorophenyl)-5-(trans-4′-propylcyclohexyl)-1,3-dioxane and 2.2 g (0.024 mol) of CuCN were refluxed with a mantle heater for two hours. The reaction product was cooled to room temperature. The cooled reaction product and 2.5 $cm^3$ of EDA added thereto were poured into 60 $cm^3$ of iced water. The resultant mixture was extracted with chloroform, washed sequentially with an aqueous EDA solution and water, and distilled to expel chloroform. The residue was dissolved in hexane, washed with water to expel the engulfed NMP, and distilled to expel hexane. The residue was re-crystallised once from a mixed solvent of acetone and methanol, treated with silica gel column using chloroform as a solvent, and distilled to expel chloroform. The residue was repeatedly re-crystallised from a mixed solvent of acetone and methanol until the cis form ceased to remain. Consequently, there was obtained 1.8 g (0.005 mol) of trans-2-(4′-cyano-3′-fluorophenyl)-5-(trans-4′-propylcyclohexyl)-1,3-dioxane. During the course of reflux, decomposition of raw materials and transition of the trans form to the cis form occurred as secondary reactions. When this compound was tested for phase transition point with DSC, the following results were obtained.

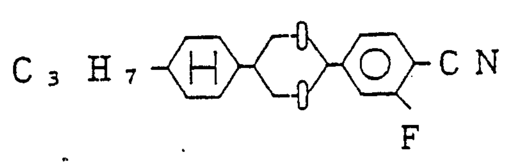

$$C \xrightarrow{76.7\,^{\circ}C} N \xrightarrow{176.8\,^{\circ}C} I$$

The following compounds were produced by following the procedure of Example 5.

Trans-2-(4′-cyano-3′-fluorophenyl)-5-(trans-4′-methylcyclohexyl)-1,3-dioxane,
Trans 2-(4′-cyano-3′-fluorophenyl)-5-(trans-4′-ethylcyclohexyl)-1,3-dioxane,

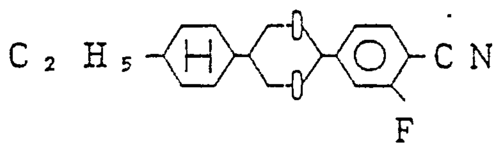

$$C \xrightarrow{70.5^{\circ}C} N \xrightarrow{145.9^{\circ}C} I$$

Trans-2-(4′-cyano-3′-fluorophenyl)-5-(trans-4′-butylcyclohexyl)-1,3-dioxane,

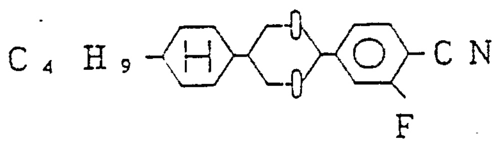

$$C \xrightarrow{89.6^{\circ}C} N \xrightarrow{175.1^{\circ}C} I$$

Trans-2-(4′-cyano-3′-fluorophenyl)-5-(trans-4′-pentylcyclohexyl)-1,3-dioxane,

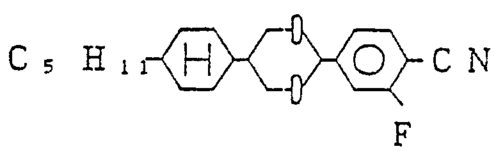

$$C \xrightarrow{67.1^{\circ}C} N \xrightarrow{178.3^{\circ}C} I$$

Trans-2-(4′-cyano-3′-fluorophenyl)-5-(trans-4′-hexylcyclohexyl)-1,3-dioxane,

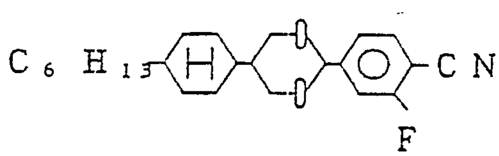

$$C \xrightarrow{67.0^{\circ}C} N \xrightarrow{170.8^{\circ}C} I$$

Trans-2-(4′-cyano-3′-fluorophenyl)-5-(trans-4′-heptylcyclohexyl)-1,3-dioxane,
Trans-2-(4′-cyano-3′-fluorophenyl)-5-(trans-4′-octylcyclohexyl)-1,3-dioxane,
Trans-2-(4′-cyano-3′-fluorophenyl)-5-(trans-4′-nonylcyclohexyl)-1,3-dioxane,
Trans-2-(4′-cyano-3′-fluorophenyl)-5-(trans-4′-decylcyclohexyl)-1,3-dioxane.

Example 6. Method for production of 2-[2′(trans-4′-butylcyclohexyl)-ethyl]propane-1,3-diol (Scheme 6).

Step 6-1: In 200 $cm^3$ of toluene, 55 g (0.30 mol) of trans-4-butylcyclohexane carboxylic acid was dispersed and stirred and 250 $cm^3$ (0.90 mol) of 70% $NaAlH_2(OC_2H_4OCH_3)_2$ solution in toluene was added dropwise thereto at a rate enough for toluene to reflux gently. After the dropwise addition was completed, the resultant mixture was stirred over a hot water bath at 90 °C for three hours. The reaction solution was cooled to room temperature and kept stirred and, at the same time, 10 $cm^3$ of water and 300 $cm^3$ of an aqueous 15% hydrochloric acid solution were added thereto dropwise. The oily layer was separated and the water layer was extracted with toluene. The two layers were joined and then washed with an aqueous 10% hydrochloric acid solution and water. The washed mixture was distilled to expel toluene. The residual oily substance was distilled under a vacuum (b.p. 100 °C/2 mmHg), to obtain 37 g (0.11 mol) of trans-4-butylcyclohexyl methanol.

An alternative method which produced trans-4-butylcyclohexyl methanol by chlorinating trans-4-butylcyclohexane carboxylic acid thereby preparing trans-4-butylcyclohexanecarbonyl chloride and reducing the product of chlorination with $NaAlH_2(OC_2H_4OCH_3)_2$ will be described below.

Over a hot water bath, 55 g (0.30 mol) of trans4-butylcyclohexane carboxylic acid and 53 g (0.45 mol) of $SOCl_2$ were refluxed and then distilled under a vacuum to expel excess $SOCl_2$. A solution prepared by dissolving 110 $cm^3$ (0.39 mol) of 70% $NaAlH_2(OC_2H_4OCH_3)_2$ solution in toluene in 110 $cm^3$ of anhydrous toluene was kept stirred and the oily substance remaining after the vacuum distillation mentioned above was added dropwise thereto at a rate enough for toluene to reflux gently. After the dropwise addition was completed, the procedure of Step 6-1 was repeated, to produce 44 g (0.26 mol) of trans-4-butylcyclohexyl methanol.

Although this method required one more reaction step, it enjoyed smaller consumption of the reducing agent and higher yield of product.

Step 6-2: Over an iced water bath, 37 g (0.22 mol) of trans-4-butylcyclohexyl methanol and 18 g (0.23 mol) of anhydrous pyridine were kept stirred and 32 g (0.27 mol of $SOCl_2$ was added dropwise thereto. The resultant reaction product was stirred with a mantle heater at 105 °C to 110 °C for three hours. The reaction product was cooled to room temperature and poured into a beaker containing concentrated hydrochloric acid and ice. The oily layer was separated and the water layer was extracted with chloroform. The two layers were joined and washed with aqueous 10% hydrochloric acid and water. The washed mixture was distilled to expel chloroform. By distilling the residual oily substance under a vacuum (b.p. 80 °C/3 mmHg), there was obtained 38 g (0.20 mol) of trans-4-butylcyclohexylchloromethane.

Step 6-3: In 40 $cm^3$ of DMSO, 38 g (0.20 mol) of trans-4-butylcyclohexylchlomomethane and 12 g (0.24 mol) of NaCN were kept stirred and heated with a mantle heater to 140 °C. The reaction product was cooled to room temperature, combined with 100 $cm^3$ of water, extracted with chloroform and washed with water. The washed reaction product was distilled to expel chloroform. The residual oily substance was subjected to vacuum distillation (b.p. 90 °C/3 mmHg), to produce 34 g (0.19 mol) of trans-4-butylcyclohexyl acetonitrile.

Step 6-4: A solution of 34 g (0.19 mol) of trans-4-butylcyclohexyl acetonitrile, 43 g (0.76 mol) of KOH, and 34 $cm^3$ of water in 190 $cm^3$ of ethanol was refluxed over a hot water bath until the generation of ammonia gas ceased. The reflux time was about ten hours and the detection of ammonia gas was carried out with a phenolphthalein test paper. The reaction solution was cooled to room temperature and distilled to expel ethanol. The residue was dissolved in 100 $cm^3$ of water. The resultant solution was poured into a beaker containing 100 g of water and 100 $cm^3$ of concentrated hydrochloric acid. The crystals which were consequently precipitated in the beaker were separated by filtration and washed with water. The crystals were re-crystallised from a mixed solvent of methanol and water, to obtain 37 g (0.19 mol) of trans-4-butylcyclohexyl acetic acid.

Step 6-5: In 190 $cm^3$ of anhydrous toluene, 37 g (0.19 mol) of trans-4-butylcyclohexyl acetic acid was dispersed and kept stirred and 160 $cm^3$ (0.57 mol) of a 70% $NaAlH_2(OC_2H_4OCH_3)_2$ solution in toluene was added dropwise thereto at a rate enough for toluene to reflux gently. After the dropwise addition was completed, the resultant mixture was kept stirred and 40 $cm^3$ of water and 500 $cm^3$ of an aqueous 15% hydrochloric acid solution were added dropwise thereto. The oily layer was separated and the water layer was extracted with toluene. The two layers were combined and washed with an aqueous 10% hydrochloric acid solution and water. The washed mixture was distilled to expel toluene. The residual oily substance was subjected to vacuum distillation (b.p. 103 °C/3 mmHg), to obtain 31 g (0.17 mol) of 2-(trans-4′-butylcyclohexyl)ethanol. Alternatively, the same product was obtained in a high yield by chlorinating trans-4-butylcyclohexyl acetic acid with $SOCl_2$ thereby preparing trans-4-butylcyclohexylacetyl chloride and reducing the product of chlorination with $NaAlH_2(OC_2H_4OCH_3)_2$.

Step 6-6: A mixture consisting of 31 g (0.17 mol) of 2-(trans-4′-butylcyclohexyl)ethanol and 14 g (0.18 mol) of anhydrous pyridine was kept stirred over an iced water bath and 25 g (0.21 mol) of $SOCl_2$ was

added dropwise thereto. The resultant reaction product was stirred with a mantle heater at 105 °C to 110 °C for three hours. The reaction product was cooled to room temperature and poured into a beaker containing ice and concentrated hydrochloric acid. The oily layer was separated and the water layer was extracted with chloroform. The two layers were combined and then washed with aqueous 10% hydrochloric acid and water. The washed mixture was distilled to expel chloroform. The residual liquid substance was subjected to distillation under a vacuum (b.p. 96 °C/3 mmHg), to obtain 30 g (0.15 mol) of 2-(trans-4′-butylcyclohexyl)-1-chloroethane.

Step 6-7: A solution of 3.5g (0.15 mol) of sodium in 150 $cm^3$ of anhydrous ethanol and 29 g (0.18 mol) of diethyl malonate and 30 g (0.15 mol) of 2-(trans-4′-butylcyclohexyl)-1-chloroethane added thereto were refluxed over a hot water bath for ten hours. The resultant reaction solution was cooled to room temperature. NaCl formed by the reaction was separated by filtration. The filtrate was distilled to expel ethanol. The residue was combined with water, extracted with chloroform, and washed with water, aqueous 10% hydrochloric acid, and water sequentially in the order mentioned. The washed residue was distilled to expel chloroform. The residual liquid substance was subjected to vacuum distillation (b.p. 165 °C/3 mmHg), to obtain 29 g (0.08 mol) of diethyl 2-(trans-4′-butylcyclohexyl)ethyl malonate. The product was obtained in a yield of 76% when the procedure was repeated, excepting 2-(trans-4′-butylcyclohexyl)-1-bromoethane was used in the place of 2-(trans-4′-butylcyclohexyl)-1-chloroethane.

Step 6-8: A solution of 130 $cm^3$ (0.48 mol) of a toluene 70% $NaAlH_2(OC_2H_4OCH_3)_2$ solution in 130 $cm^3$ of anhydrous toluene was kept stirred and 29 g (0.08 mol) of diethyl 2-(trans-4′-butylcyclohexyl)ethyl malonate was added dropwise thereto. After the dropwise addition was completed, the resultant reaction mixture was heated over a hot water bath at 80 to 90 °C for five hours. The resultant reaction product was cooled to room temperature and 30 $cm^3$ of water and 400 $cm^3$ of an aqueous 15% hydrochloric acid solution were added dropwise thereto while continuously stirring. The oily layer was separated and the water layer was extracted with toluene. The two layers were combined and washed with an aqueous 10% hycrochloric acid solution and water. The washed mixture was distilled to expel toluene. The residue was re-crystallised from hexane, to obtain 14 g (0.06 mol) of 2-[2′(trans-4′-butylcyclohexyl)ethyl]propane-1,3-diol.

<u>Example 7.</u> Method for production of trans-2-(4′-fluorophenyl)-5-[2′-(trans-4′-butylcyclohexyl)ethyl]-1,3-dioxane (Scheme 7)

Step 7-1: A solution of 2.4 g (0.01 mol) of 2-[2′(trans-4′-butylcyclohexyl)ethyl]propane-1,3-diol, 1.8 g (0.01 mol) of 4-fluorobenzaldehyde, and 0.1 g of TsOH in 50 $cm^3$ of dichloromethane was refluxed with the aid of a Dean-Stark trap for three hours. The resultant reaction solution was washed with water and then distilled to expel dichloromethane. The residue of distillation was re-crystallised from a mixed solvent of acetone and methanol, to obtain 3.0 g (0.008 mol) of trans-2-(4′-fluorophenyl)-5-[2′-(trans-4′-butylcyclohexyl)ethyl]-1,3-dioxane. This compound was tested for phase transition point by the use of DSC. The results were as shown below.

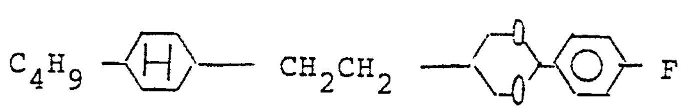

$$C \xrightarrow{73.0\ °C} N \xrightarrow{104.8\ °C} I$$

The following compounds were produced by following the procedure of Example 7.

Trans-2-(4′-fluorophenyl)-5-[2′-(trans-4′-methylcyclohexyl)-ethyl]-1,3-dioxane,
Trans-2-(4′-fluorophenyl)-5-[2′-(trans-4′-ethylcyclohexyl)-ethyl]-1,3-dioxane,
Trans-2-(4′-fluorophenyl)-5-[2′-(trans-4′-propylcyclohexyl)-ethyl]-1,3-dioxane,

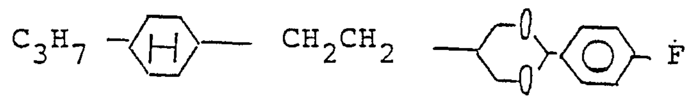

$C \xrightarrow{75.4\ ^\circ C} N \xrightarrow{116.7\ ^\circ C} I$

Trans-2-(4′-fluorophenyl)-5-[2′-(trans-4′-pentylcyclohexyl)-ethyl]-1,3-dioxane,

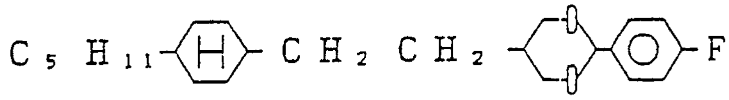

$C \xrightarrow{69.7\ ^\circ C} N \xrightarrow{117.9\ ^\circ C} I$

$S \xleftarrow{59.3\ ^\circ C} N$

Trans-2-(4′-fluorophenyl)-5-[2′-(trans-4′-hexylcyclohexyl)-ethyl]-1,3-dioxane,

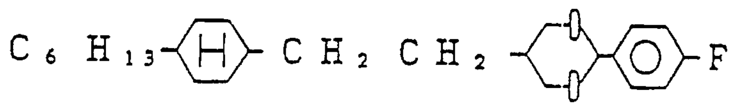

$C \xrightarrow{77.4\ ^\circ C} N \xrightarrow{113.5\ ^\circ C} I$

Trans-2-(4′-fluorophenyl)-5-[2′-(trans-4′-heptylcyclohexyl)-ethyl]-1,3-dioxane,
Trans-2-(4′-fluorophenyl)-5-[2′-(trans-4′-octylcyclohexyl)-ethyl]-1,3-dioxane,
Trans-2-(4′-fluorophenyl)-5-[2′-(trans-4′-nonylcyclohexyl)-ethyl]-1,3-dioxane,
Trans-2-(4′-fluorophenyl)-5-[2′-(trans-4′-decylcyclohexyl)-ethyl]-1,3-dioxane.

Example 8. Method for production of trans-2-(3′,4′-difluorophenyl)-5-[2′-(trans-4″-butylcyclohexyl)-ethyl]-1,3-dioxane (Scheme 8)

Step 8-1: A solution of 2.4 g (0.01 mol) of 2-[2′-(trans-4″-butylcyclohexyl)-ethyl]propane-1,3-diol, 2.8 g (0.01 mol) of 3,4-difluorobenzaldehyde, and 0.1 g of TsOH in 50 $cm^3$ of dichloromethane was refluxed with the aid of a Dean-Stark trap for three hours. The reaction solution was washed with water and distilled to expel dichloromethane. The residue was re-crystallised from a mixed solvent of acetone and methanol to obtain 3.0 g (0.008 mol) of trans-2-(3′,4′-difluorophenyl)-5-[2′-(trans-4″-butylcyclohexyl)-ethyl]-1,3-dioxane. This compound was tested for phase transition point by the use of DSC. The results were as shown below.

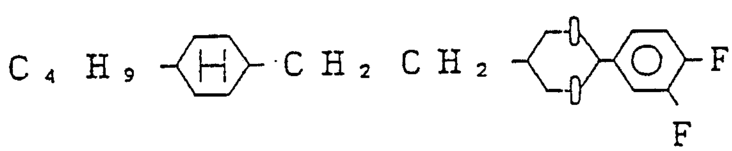

$C \xrightarrow{83.0\ ^\circ C} N \xrightarrow{87.0\ ^\circ C} I$

The following compounds were produced by following the procedure of Example 8.
Trans-2-(3′,4′-difluorophenyl)-5-[2′-(trans-4″-methylcyclohexyl)-ethyl]-1,3-dioxane,
Trans-2-(3′,4′-difluorophenyl)-5-[2′-(trans-4″-ethylcyclohexyl)-ethyl]-1,3-dioxane,
Trans-2-(3′,4′-difluorophenyl)-5-[2′-(trans-4″-propylcyclohexyl)-ethyl]-1,3-dioxane,

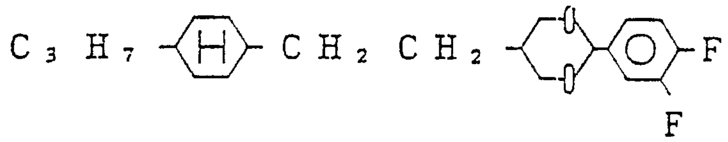

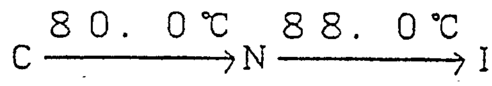


Trans-2-(3′,4′-difluorophenyl)-5-[2′-(trans-4″-pentylcyclohexyl)-ethyl]-1,3-dioxane,

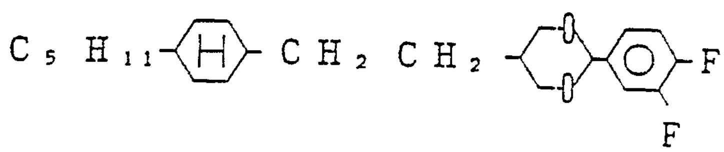

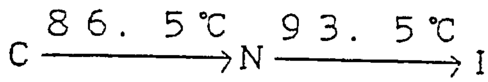


Trans-2-(3′,4′-difluorophenyl)-5-[2′-(trans-4″-hexylcyclohexyl)-ethyl]-1,3-dioxane,

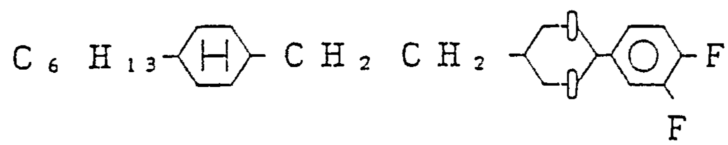

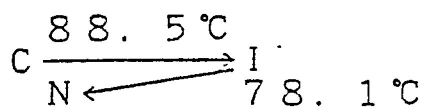


Trans-2-(3′,4′-difluorophenyl)-5-[2′-(trans-4″-heptylcyclohexyl)-ethyl]-1,3-dioxane,
Trans-2-(3′,4′-difluorophenyl)-5-[2′-(trans-4″-octylcyclohexyl)-ethyl]-1,3-dioxane,
Trans-2-(3′,4′-difluorophenyl)-5-[2′-(trans-4″-nonylcyclohexyl)-ethyl]-1,3-dioxane,
Trans-2-(3′,4′-difluorophenyl)-5-[2′-(trans-4″-decylcyclohexyl)-ethyl]-1,3-dioxane.

Example 9. Method for production of trans-2-(4′-cyano-3′-fluorophenyl)-5-[2′-(trans-4″-butylcyclohexyl)-ethyl]-1,3-dioxane (Scheme 9)

Step 9-1: A solution of 6.1 g (0.025 mol) of 2-[2′-(trans-4″-butylcyclohexyl)-ethyl]propane-1,3-diol, 5.1 g (0.025 mol) of the 4-bromo-3-fluorobenzaldehyde obtained in Example 4, and 0.1 g of TsOH in 125 $cm^3$ of dichloro-methane was refluxed with the aid of a Dean-Stark trap for five hours. The reaction solution was washed with water and distilled to expel dichloromethane. The residue was re-crystallised from a mixed solvent of acetone and methanol, to obtain 8.4 g (0.02 mol) of 2-(4′-bromo-3′-fluorophenyl)-5-[2′-(trans-4″-butylcyclo-hexyl)-ethyl]-1,3-dioxane.

Step 9-2: In 100 $cm^3$ of NMP, 8.4 g (0.02 mol) of 2-(4′-bromo-3′-fluorophenyl)-5-[2′-(trans-4″-butylcyclohexyl)-ethyl]-1,3-dioxane and 2.7 g (0.03 mol) of CuCN were refluxed for two hours. The reaction product was cooled to room temperature. The cooled reaction product and 4 $cm^3$ of EDA added thereto were poured into 100 $cm^3$ of iced water, extracted with hexane, and washed with an aqueous EDA solution and water. The washed mixture was distilled to expel hexane. The residue was treated with a silica gel column using chloroform as a solvent and the eluate was distilled to expel chloroform. The residue was re-crystallised from a mixed solvent of acetone and methanol, to obtain 3.1 g (0.008 mol) of trans-2-(4′-cyano-3′-fluorophenyl)-5-[2′-(trans-4″-butylcyclohexyl)-ethyl]-1,3-dioxane. This compound was tested for phase

transition point by the use of DSC. The results were as shown below.

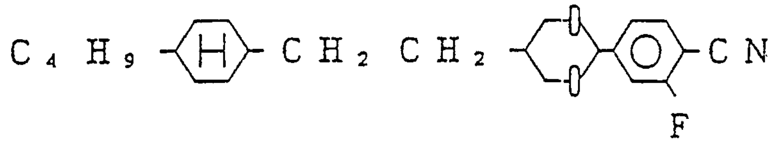

$$C \xrightarrow{63.5^{\circ}C} N \xrightarrow{142.8^{\circ}C} I$$

The following compounds were produced by following the procedure of Example 9.

Trans-2-(4′-cyano-3′-fluorophenyl)-5-[2′-(trans-4″-methylcyclohexyl)-ethyl]-1,3-dioxane,

Trans-2-(4′-cyano-3′-fluorophenyl)-5-[2′-(trans-4″-ethylcyclohexyl)-ethyl]-1,3-dioxane,

Trans-2-(4′-cyano-3′-fluorophenyl)-5-[2′-(trans-4″-propylcyclohexyl)-ethyl]-1,3-dioxane,

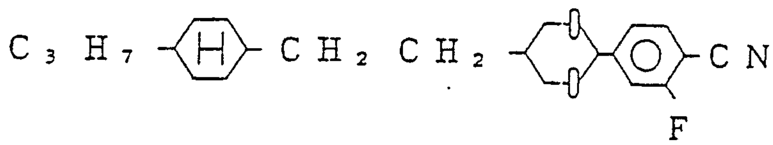

$$C \xrightarrow{91.0^{\circ}C} N \xrightarrow{145.4^{\circ}C} I$$

Trans-2-(4′-cyano-3′-fluorophenyl)-5-[2′-(trans- 4″-pentylcyclohexyl)-ethyl]-1,3-dioxane,

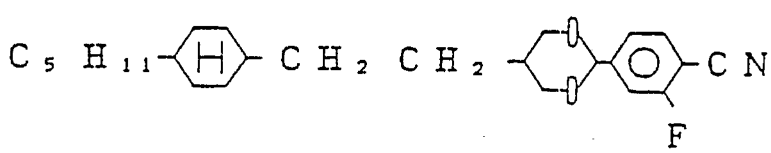

$$C \xrightarrow{85.2^{\circ}C} N \xrightarrow{145.0^{\circ}C} I$$

Trans-2-(4′-cyano-3′-fluorophenyl)-5-[2′-(trans-4″-hexylcyclohexyl)-ethyl]-1,3-dioxane,

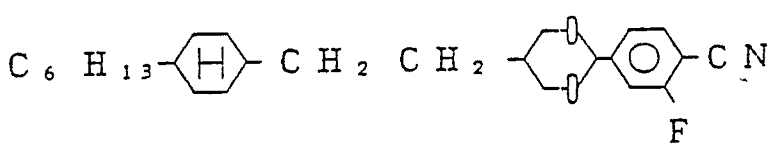

$$C \xrightarrow{90.0^{\circ}C} N \xrightarrow{136.1^{\circ}C} I$$

Trans-2-(4′-cyano-3′-fluorophenyl)-5-[2′-(trans-4″-heptylcyclohexyl)-ethyl]-1,3-dioxane,

Trans-2-(4′-cyano-3′-fluorophenyl)-5-[2′-(trans-4″-octylcyclohexyl)-ethyl]-1,3-dioxane,

Trans-2-(4′-cyano-3′-fluorophenyl)-5-[2′-(trans-4″-nonylcyclohexyl)-ethyl]-1,3-dioxane,

Trans-2-(4′-cyano-3′-fluorophenyl)-5-[2′-(trans-4″-decylcyclohexyl)-ethyl]-1,3-dioxane.

Example 10. Method for production of trans-2-(4′-cyanophenyl)-5-(trans-4′-butylcyclohexyl)-1,3-dioxane (Scheme 10)

Step 10-1: A solution of 4.6 g (0.02 mol of 2-(4′-butylcyclohexyl)propane-1,3-diol, 3.2 g (0.024 mol) of 4-cyanobenzaldehyde and 0.2 g of TsOH in dichloromethane was refluxed for three hours over a hot water bath fitted with a Dean-Stark trap, with the water formed continuously removed from the reaction system. The reaction solution was washed with water and distilled to expel dichloromethane. By re-crystallising the residue of distillation from a mixed solvent of acetone and methanol, there was obtained 1.4 g (0.004 mol) of trans-2-(4′-cyanophenyl)-5-(trans-4′-butylcyclohexyl)-1,3-dioxane. This compound was tested for phase transition point by the use of DSC. The results were as shown below.

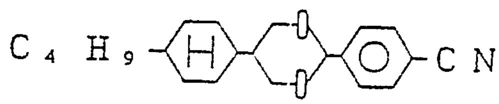

$$C \xrightarrow{74.6℃} S \xrightarrow{91.5℃} N \xrightarrow{221.2℃} I$$

The following compounds were produced by following the procedure of Example 10.
Trans-2-(4′-cyanophenyl)-5-(trans-4′-ethylcyclohexyl)-1,3-dioxane,

$$C \xrightarrow{116.5°C} N \xrightarrow{205.4°C} I$$

Trans-2-(4′-cyanophenyl)-5-(trans-4′-propylcyclohexyl)-1,3-dioxane,

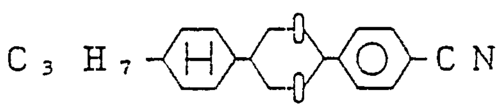

$$C \xrightarrow{89.5℃} N \xrightarrow{222.7℃} I$$

Trans-2-(4′-cyanophenyl)-5-(trans-4′-pentylcyclohexyl)-1,3-dioxane,

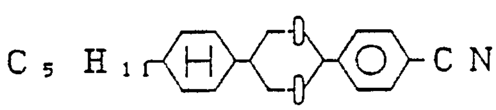

$$C \xrightarrow{87.0℃} N \xrightarrow{222.1℃} I$$

Trans-2-(4′-cyanophenyl)-5-(trans-4′-hexylcyclohexyl)-1,3-dioxane,

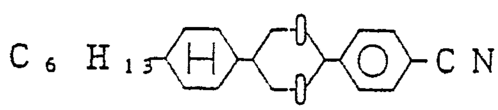

$$C \xrightarrow{40.4℃} S_1 \xrightarrow{55.9℃} S_2 \xrightarrow{95.6℃} N \xrightarrow{213.9℃} I$$

Example 11 Method for production of trans-2-(4′-cyanophenyl)-5-[2′-(trans-4″-butylcyclohexyl)ethyl]-1,3-dioxane (Scheme 11)

Step 11-1: A solution of 4.9 g (0.02 mol) of 2-[2′-(trans-4′-butylcyclohexyl)ethyl]propane-1,3-diol, 3.2 g (0.024 mol) of 4-cyanobenzaldehyde, and 0.2 g of TsOH in 100 $cm^3$ of dichloromethane was refluxed for three hours over a hot water bath fitted with a Dean-Stark trap, with the formed water continuously removed from the reaction system. The reaction solution was washed with water and distilled to expel dichloromethane. The residue was re-crystallised from a mixed solvent of acetone and methanol, to obtain 4.9 g (0.014 mol) of trans-2-(4′-cyanophenyl)-5-[2′-(trans-4″-butylcyclohexyl)ethyl]-1,3-dioxane. This compound was tested for phase transition point by the use of DSC. The results were as shown below.

The following compounds were produced by following the procedure of Example 11.

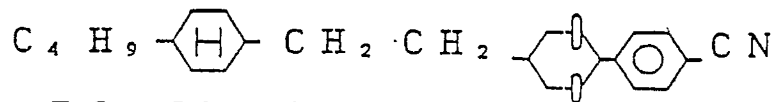

$$C \xrightarrow{72.2^\circ C} S \xrightarrow{91.7^\circ C} N \xrightarrow{179.4^\circ C} I$$

Trans-2-(4′-cyanophenyl)-5-[2′-(trans-4″-propylcyclohexyl)ethyl]-1,3-dioxane,

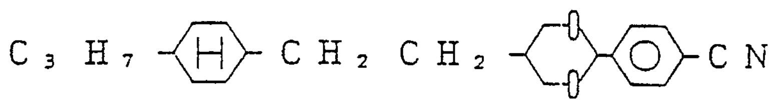

$$C \xrightarrow{84.3^\circ C} N \xrightarrow{183.2^\circ C} I$$

Trans-2-(4′-cyanophenyl)-5-[2′-(trans-4″-pentylcyclohexyl)ethyl]-1,3-dioxane,

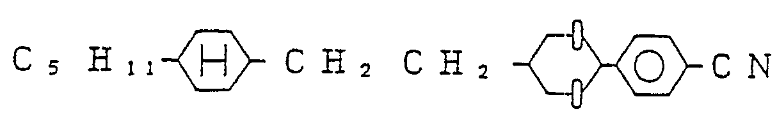

$$C \xrightarrow{96.8^\circ C} N \xrightarrow{180.6^\circ C} I$$

Trans-2-(4′-cyanophenyl)-5-[2′-(trans-4″-hexylcyclohexyl)ethyl]-1,3-dioxane,

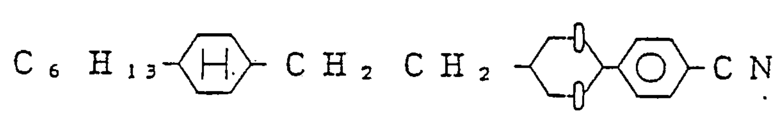

$$C \xrightarrow{96.9^\circ C} N \xrightarrow{172.0^\circ C} I$$

Example 12

Liquid crystal compositions [A-1], [B-1], [C-1], [D-1], [E-1], and [F-1] were prepared by mixing three compounds each of the types (1-a), (1-b), (1-c), (1-d), (1-e) and (1-f) of the present invention severally having a propyl group, a butyl group, and a pentyl group as the substituent R in a ratio of 1 : 1 : 1. For comparison, liquid crystal compositions (K-1) and (II-1) were prepared by mixing three compounds each of the types (I) and (II) severally having a propyl group, a butyl group and a pentyl group as the substituent R in a ratio of 1 : 1 : 1.

Liquid crystal composition [A-1]

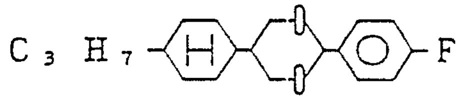 33.3 WT %

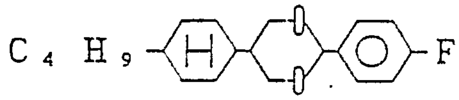 33.3 WT %

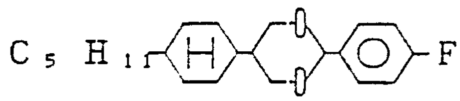 33.3 WT %

Liquid crystal composition [B-1]

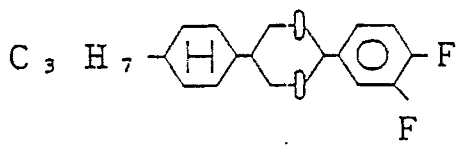 33.3 WT %

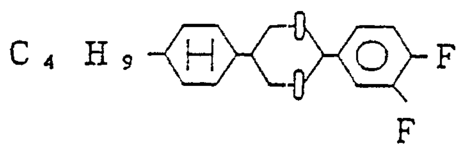 33.3 WT %

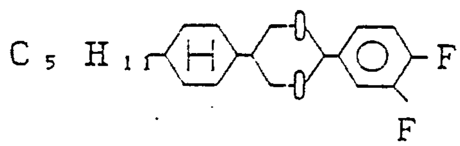 33.3 WT %

Liquid crystal composition [C-1]

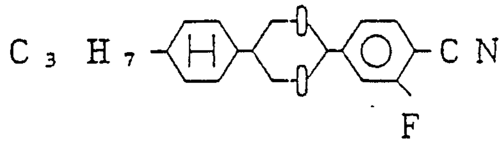 33.3 WT %

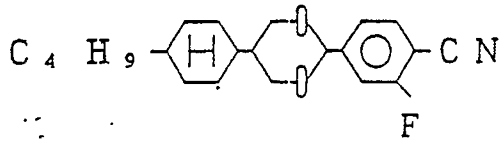 33.3 WT %

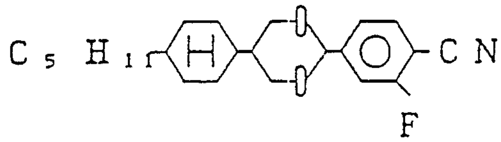 33.3 WT %

Liquid crystal composition [D-1]

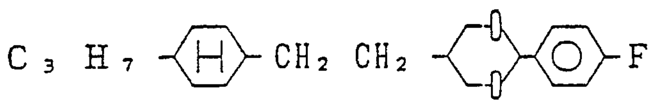 33.3 WT %

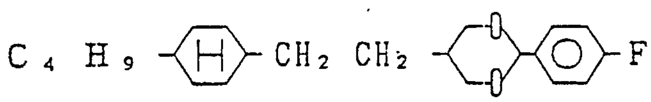 33.3 WT %

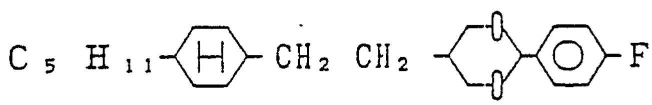 33.3 WT %

Liquid crystal composition [E-1]

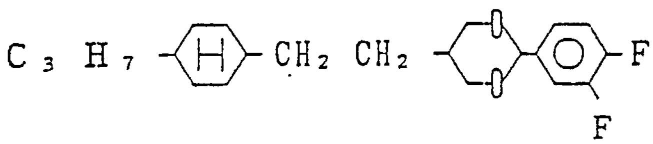 33.3 WT %

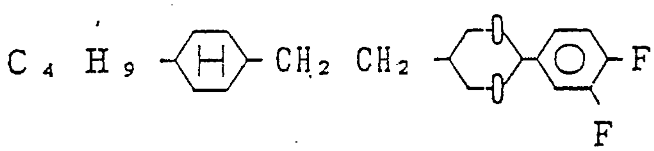 33.3 WT %

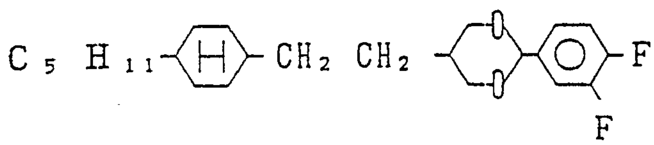 33.3 WT %

Liquid crystal composition [F-1]

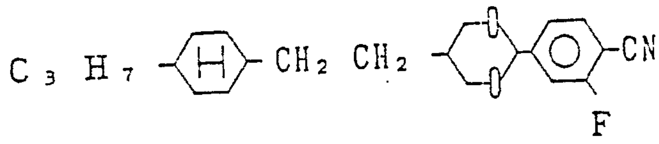
33.3 WT %

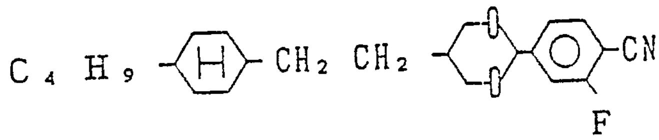
33.3 WT %

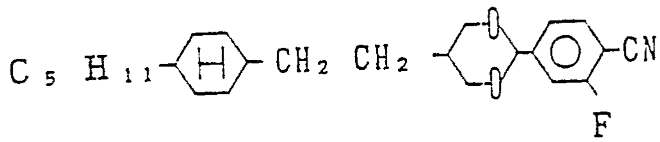
33.3 WT %

Liquid crystal composition [I-1]

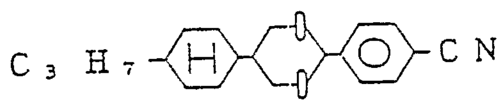
33.3 WT %

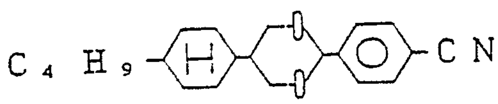
33.3 WT %

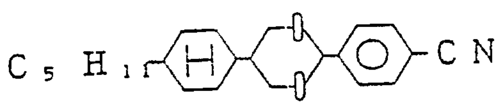
33.3 WT %

Liquid crystal composition [II-1]

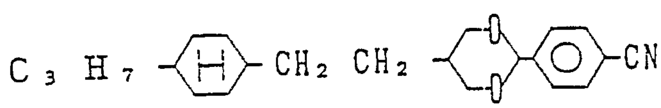
33.3 WT %

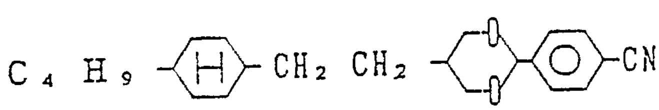
33.3 WT %

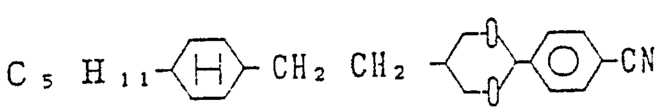
33.3 WT %

Further liquid crystal compositions were prepared by mixing the eight liquid crystal compositions prepared as described above severally in the varying mixing ratios of 10, 20, 30, 40 and 50% by weight with a commercially available liquid crystal composition (produced by Merck, and marketed under the product code of "ZLI-1565"). They were left standing at -20 °C for five hundred hours to determine whether or not they would form crystals in the course of the standing. The results of this test were as shown in Table 4.

Table 4

| (in % by weight) | | | | | |
|---|---|---|---|---|---|
| ZLI-1565 | 90 | 80 | 70 | 60 | 50 |
| Mixing ratio | 10 | 20 | 30 | 40 | 50 |
| [A-1] | O | O | O | × | × |
| [B-1] | O | O | O | O | × |
| [C-1] | O | O | O | × | × |
| [D-1] | O | O | O | × | × |
| [E-1] | O | O | O | O | × |
| [F-1] | O | O | O | × | × |
| [I-1] | O | O | × | × | × |
| [II-1] | O | O | × | × | × |

In this Table, 0 denotes absence of the formation of crystals and X the formation of crystals.

From the results given above, it is clearly noted that the compounds (I) and (II) used for comparison were deficient in compatibility. The compounds of this invention generally possessed satisfactory compatibility, those of the compounds involving substitution of two fluorine atoms particularly excelled in compatibility. The results indicate that 1,3-dioxane derivatives of the present invention could be incorporated in ratios up to 30% by weight in the conventional compositions.

Example 13.

Liquid crystal compositions [A-2], [B-2], [C-2], [D-2], [E-2], and [F-2] were prepared by mixing 1,3-dioxane derivatives of the present invention having $C_4H_9$ as the substituent R each with ZLI-1565. For comparison, liquid crystal compositions [I-2], [II-2], [III-2] and [IV-2] were prepared severally containing the following compounds in a ratio of 10% by weight.

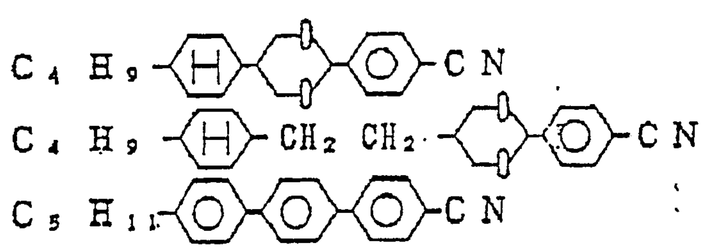

(I)

(II)

(III)

and

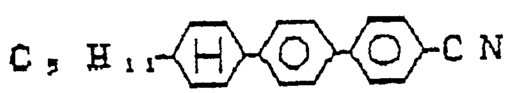

(IV)

Liquid crystal composition [A-2]

ZLI-1565 90 WT %

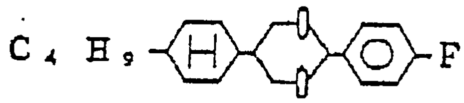 10 WT %

Liquid crystal composition [B-2]

ZLI-1565 90 WT %

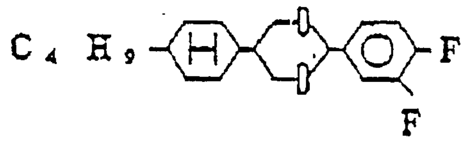 10 WT %

Liquid crystal composition [C-2]

ZLI-1565 90 WT %

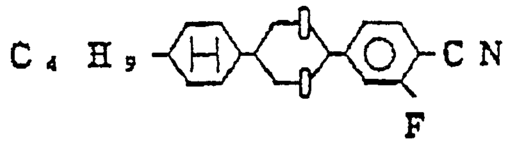 10 WT %

Liquid crystal composition [D-2]

ZLI-1565 90 WT %

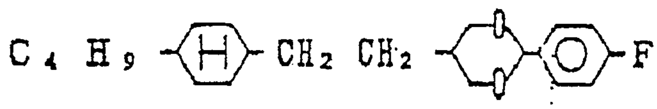 10 WT %

Liquid crystal composition [E-2]

ZLI-1565 90 WT %

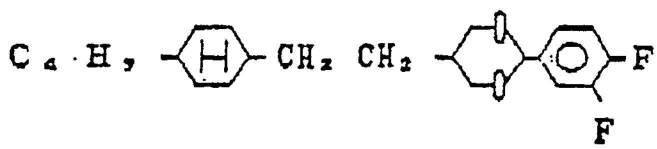 10 WT %

Liquid crystal composition [F-2]

ZLI-1565 90 WT %

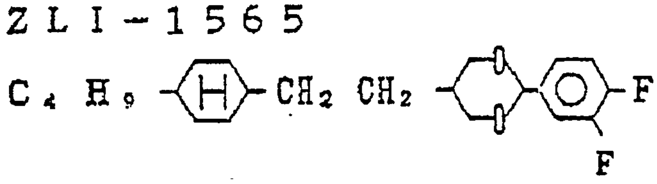 10 WT %

Liquid crystal composition [I-2]

ZLI-1565 90 WT %

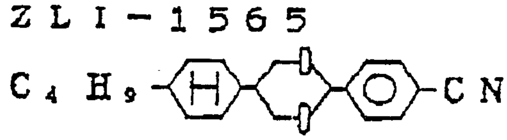 10 WT %

Liquid crystal composition [II-2]

| | | |
|---|---|---|
| ZLI-1565 | 90 | WT % |
| 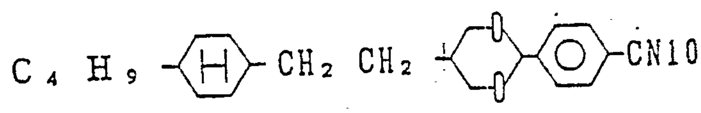 | 10 | WT % |

Liquid crystal composition [III-2]

| | | |
|---|---|---|
| ZLI-1565 | 90 | WT % |
| 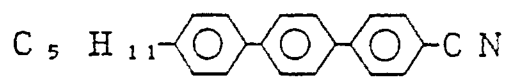 | 10 | WT % |

Liquid crystal composition [IV-2]

| | | |
|---|---|---|
| ZLI-1565 | 90 | WT % |
| 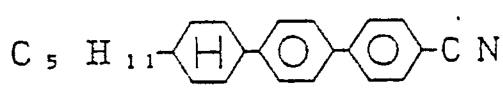 | 10 | WT % |

These liquid crystal compositions were tested for N-I point and $\Delta n$. They were each sealed in a TN type cell and tested at 20°C for threshold voltage ($V_{th}$) of voltage luminance characteristic, dependency on visual angle ($\alpha$), precipitousness ($\gamma$), initiating speed ($\tau_{ON}$), trailing speed ($\tau_{OFF}$), and at a varying temperature in the range of 0° to 40°C for dependency on temperature ($\Delta T$) and voltage margin (M). $V_{th}$ and $V_{sat}$ were taken as voltages at 10% and 90% (50% for M) of luminance in the V-$I_0$ curve. Here, $\alpha$, $\gamma$, $\Delta T$ and M were defined by the following formulae:

$$\alpha = \frac{V_{th}(T = 20℃、\phi = 10°)}{V_{th}(T = 20℃、\phi = 40°)}$$

$$\gamma = \frac{V_{sat}(T = 20℃、\phi = 10°)}{V_{th}(T = 20℃、\phi = 10°)}$$

$$\Delta T = V_{th}(T=0℃、\phi=10°) - V_{th}(T=40℃、\phi=10°)$$

$$M = \frac{V_{th}(T=40℃、\phi=40°) - V_{sat}(T=0℃、\phi=10°)}{V_{th}(T=40℃、\phi=40°) + V_{sat}(T=0℃、\phi=10°)} \times 100$$

In the formulae, T stands for temperature, $\phi$ for visual angle (relative to the front side taken as 0°), and M for a value at 1/3 bias - 1/3 duty.

The results of the test were as shown collectively in Table 5.

Table 5

| Composition | N-I POINT (°C) | Δ • n | V th(V) | α | γ | τ ON(ms) | τ OFF (ms) | ΔT(V) | M(%) |
|---|---|---|---|---|---|---|---|---|---|
| A-2 | 92.1 | 0.124 | 2.51 | 1.30 | 1.45 | 58 | 124 | -0.28 | 9.2 |
| B-2 | 89.5 | 0.123 | 2.42 | 1.29 | 1.48 | 56 | 127 | -0.30 | 8.6 |
| C-2 | 93.8 | 0.130 | 2.19 | 1.29 | 1.38 | 60 | 113 | -0.31 | 9.6 |
| D-2 | 89.0 | 0.124 | 2.50 | 1.31 | 1.49 | 52 | 121 | -0.25 | 8.9 |
| E-2 | 86.5 | 0.123 | 2.38 | 1.30 | 1.47 | 54 | 125 | -0.30 | 9.5 |
| F-2 | 92.7 | 0.127 | 2.23 | 1.29 | 1.41 | 68 | 132 | -0.31 | 9.8 |
| I-2 | 99.3 | 0.130 | 2.38 | 1.30 | 1.48 | 57 | 130 | -0.31 | 8.4 |
| II-2 | 95.2 | 0.128 | 2.45 | 1.31 | 1.48 | 59 | 136 | -0.31 | 8.2 |
| III-2 | 100.8 | 0.149 | 2.52 | 1.30 | 1.49 | 49 | 102 | -0.29 | 8.2 |
| IV-2 | 99.4 | 0.139 | 2.48 | 1.32 | 1.46 | 48 | 108 | -0.29 | 8.8 |

Example 14

A liquid crystal composition [M-3] composed of Nn type phenylcyclohexane carboxylic esters and Np type cyanophenylbenzoic esters as shown below was prepared by admixture.

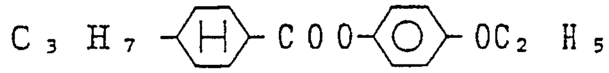 7.9 WT %

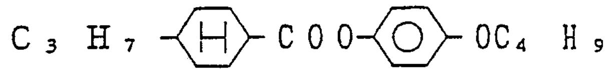 20.5 WT %

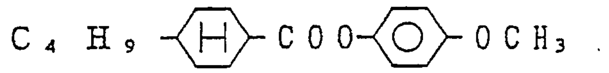 15.9 WT %

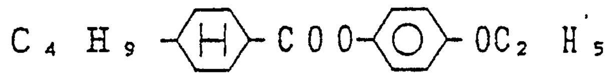 16.0 WT %

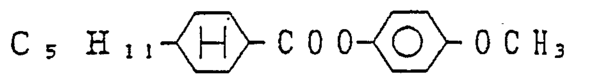 17.5 WT %

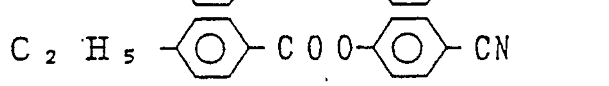 11.1 WT %

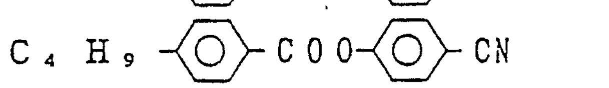 11.1 WT %

Liquid crystal compositions [A-3], [B-3], [C-3], [D-3], [E-3], [F-3], [I-3], [II-3], [III-3] and [IV-3] were prepared by mixing the liquid crystal composition [M-3] with 10% by weight each of the 1,3-dioxane derivatives according to the present invention having $C_4H_9$ as the substituent R, the compounds (I) and (II) for comparison having $C_4H_9$ as the substituent R, and the compounds (III) and (IV). They were tested for N-I point, Δn, $V_{th}$, α, γ, $\tau_{ON}$, $\tau_{OFF}$, ΔT and M in the same manner as in Example 13. The results were as shown in Table 6.

Liquid crystal composition [A-3]

[M-3] 90 WT %

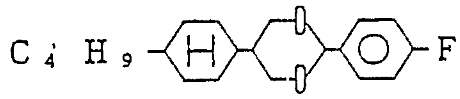 10 WT %

Liquid crystal composition [B-3]

[M-3] 90 WT %

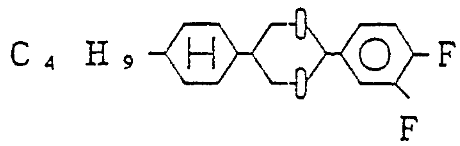 10 WT %

Liquid crystal composition [C-3]

[M-3] 90 WT %

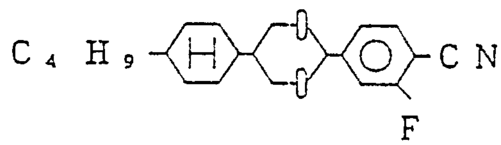 10 WT %

Liquid crystal composition [D-3]

[M-3] 90 WT %

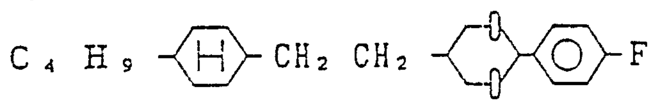 10 WT %

Liquid crystal composition [E-3]

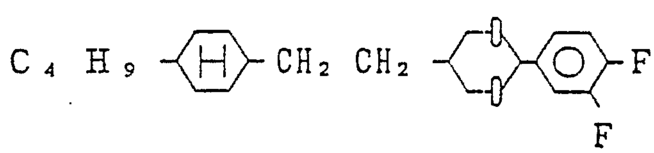 10 WT %

Liquid crystal composition [F-3]

〔M－3〕 90 WT %

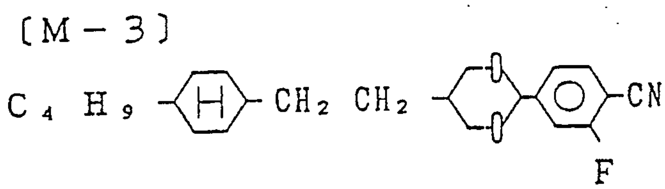
10 WT %

Liquid crystal composition [I-3]

〔M－3〕 90 WT %

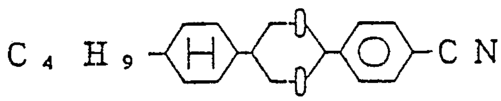
10 WT %

Liquid crystal composition [II-3]

〔M－3〕 90 WT %

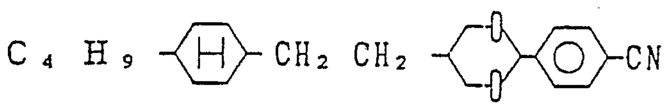
10 WT %

Liquid crystal composition [III-3]

〔M－3〕 90 WT %

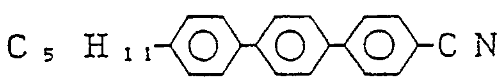
10 WT %

Liquid crystal composition [IV-3]

〔M－3〕 90 WT %

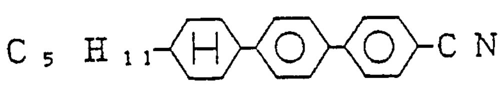
10 WT %

Table 6

| Composition | N-I POINT (°C) | Δ n | $V_{th}$(V) | α | γ | $\tau_{ON}$(ms) | $\tau_{OFF}$ (ms) | ΔT(V) | M(%) |
|---|---|---|---|---|---|---|---|---|---|
| A-3 | 66.3 | 0.101 | 1.84 | 1.25 | 1.43 | 96 | 200 | -0.30 | 10.6 |
| B-3 | 64.6 | 0.101 | 1.73 | 1.23 | 1.41 | 93 | 204 | -0.34 | 10.5 |
| C-3 | 69.3 | 0.105 | 1.68 | 1.27 | 1.45 | 100 | 211 | -0.32 | 9.3 |
| D-3 | 64.2 | 0.101 | 1.85 | 1.25 | 1.44 | 95 | 211 | -0.31 | 10.7 |
| E-3 | 63.0 | 0.101 | 1.78 | 1.23 | 1.40 | 112 | 218 | -0.34 | 10.3 |
| F-3 | 67.5 | 0.105 | 1.70 | 1.27 | 1.45 | 102 | 231 | -0.33 | 9.4 |
| I-3 | 72.0 | 0.107 | 1.78 | 1.27 | 1.43 | 99 | 210 | -0.32 | 9.5 |
| II-3 | 70.2 | 0.106 | 1.77 | 1.27 | 1.42 | 109 | 228 | -0.32 | 9.9 |
| III-3 | 73.5 | 0.127 | 1.83 | 1.28 | 1.44 | 90 | 196 | -0.26 | 10.1 |
| IV-3 | 73.9 | 0.117 | 1.89 | 1.29 | 1.45 | 85 | 189 | -0.25 | 9.8 |

## Example 15

A liquid crystal composition [M-4] composed of Nn type phenylcyclohexane carboxylic esters and Np type cyanobiphenyl and Nn type cyanophenyl pyrimidine compounds as shown below was prepared by admixture.

Liquid crystal composition [M-4]

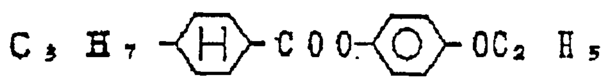 17.8 WT %

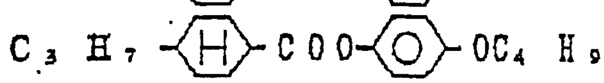 17.8 WT %

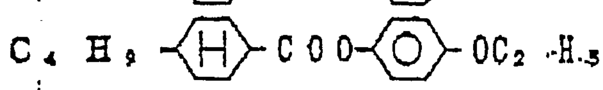 17.8 WT %

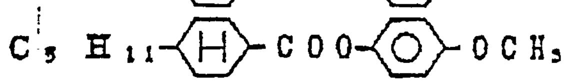 18.8 WT %

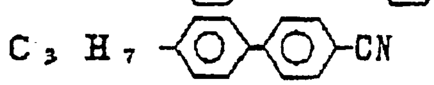 11.1 WT %

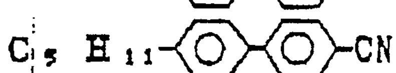 11.1 WT %

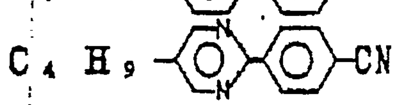 5.6 WT %

Liquid crystal compositions [A-4], [B-4], [C-4], [D-4], [E-4], [F-4], [I-4], [II-4], [III-4] and [IV-4] were prepared by following the procedure of Example 14.

Liquid crystal composition [A-4]

| | | |
|---|---|---|
| [M-4] | 90 | WT % |
| 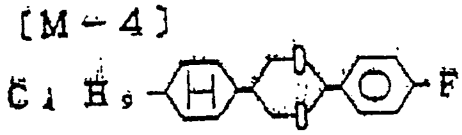 | 10 | WT % |

Liquid crystal composition [B-4]

| | | |
|---|---|---|
| [M-4] | 90 | WT % |
| 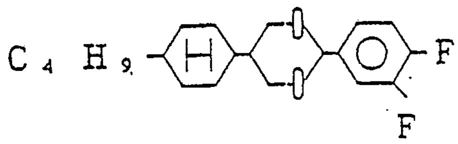 | 10 | WT % |

Liquid crystal composition [C-4]

[M-4] 90 WT %

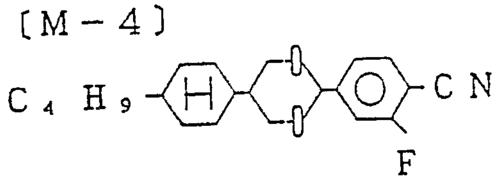 10 WT %

Liquid crystal composition [D-4]

[M-4] 90 WT %

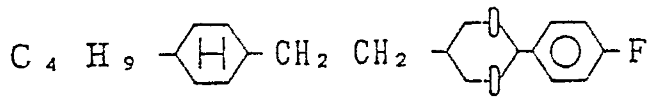 10 WT %

Liquid crystal composition [E-4]

[M-4] 90 WT %

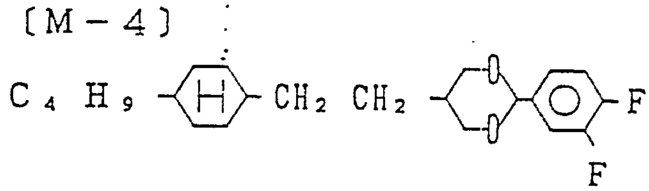 10 WT %

Liquid crystal composition [F-4]

[M-4] 90 WT %

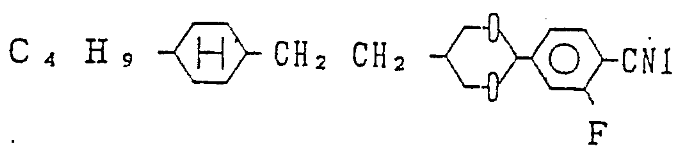 10 WT %

Liquid crystal composition [I-4]

[M-4] 90 WT %

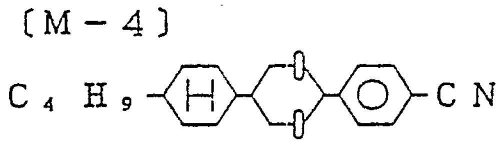 10 WT %

Liquid crystal composition [II-4]

[M-4] 90 WT %

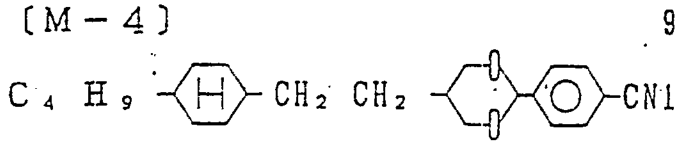 10 WT %

Liquid crystal composition [III-4]

[M-4] 90 WT %

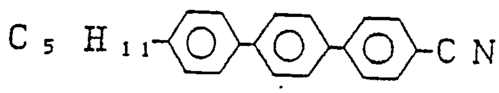 10 WT %

Liquid crystal composition [IV-4]

[M-4] 90 WT %

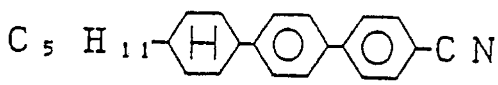 10 WT %

These liquid crystal compositions were tested for N-I point, Δn, $V_{th}$, α, γ, $\tau_{ON}$, $\tau_{OFF}$, ΔT and M. The results were as"shown collectively in Table 7.

Table 7

| Composition | N-I POINT (°C) | Δ n | $V_{th}$(V) | α | γ | $\tau_{ON}$(ms) | $\tau_{OFF}$ (ms) | ΔT(V) | M(%) |
|---|---|---|---|---|---|---|---|---|---|
| A-4 | 64.5 | 0.120 | 2.05 | 1.23 | 1.39 | 73 | 144 | -0.53 | 5.6 |
| B-4 | 62.7 | 0.118 | 1.99 | 1.22 | 1.38 | 75 | 148 | -0.55 | 4.5 |
| C-4 | 67.5 | 0.121 | 1.88 | 1.26 | 1.43 | 70 | 150 | -0.46 | 7.0 |
| D-4 | 63.2 | 0.119 | 2.03 | 1.24 | 1.42 | 66 | 140 | -0.41 | 8.3 |
| E-4 | 61.2 | 0.118 | 1.96 | 1.24 | 1.42 | 68 | 141 | -0.54 | 5.4 |
| F-4 | 66.1 | 0.123 | 1.84 | 1.25 | 1.41 | 77 | 150 | -0.42 | 8.0 |
| I-4 | 70.8 | 0.126 | 1.94 | 1.25 | 1.40 | 75 | 152 | -0.40 | 8.1 |
| II-4 | 69.1 | 0.124 | 1.99 | 1.27 | 1.44 | 72 | 154 | -0.38 | 8.3 |
| III-4 | 73.6 | 0.145 | 2.06 | 1.26 | 1.39 | 67 | 134 | -0.46 | 7.4 |
| IV-4 | 72.2 | 0.136 | 2.07 | 1.27 | 1.44 | 67 | 143 | -0.36 | 8.5 |

Example 16

A liquid crystal composition [M-5] composed of Nn type phenylcyclohexane carboxylic esters, Nn type phenylbenzoic esters, Np type cyanophenylbenzoic esters, and Np type cyanophenylcyclohexane carboxylic esters as shown below were prepared by admixture.

Liquid crystal composition [M-5]

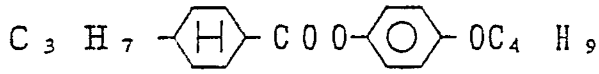 17.8 WT %

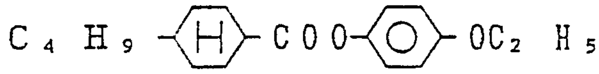 17.8 WT %

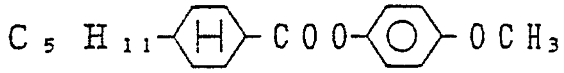 17.8 WT %

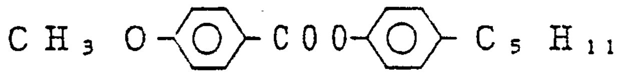 11.1 WT %

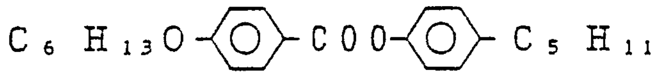 11.1 WT %

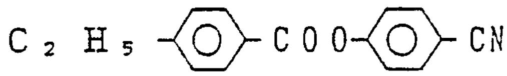 13.3 WT %

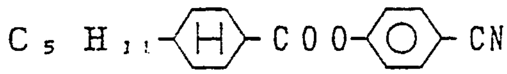 11.1 WT %

Liquid crystal compositions [A-5], [B-5], [C-5], [D-5], [E-5], [F-5], [I-5], [II-5], [III-5] and [IV-5] were prepared by following the procedure of Example 14.

Liquid crystal composition [A-5]

[M-5] 90 WT %

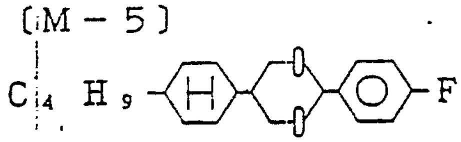 10 WT %

Liquid crystal composition [B-5]

[M-5] 90 WT %

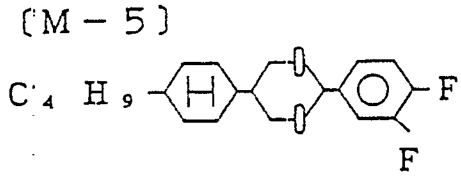 10 WT %

Liquid crystal composition [C-5]

〔M－5〕 90 WT %

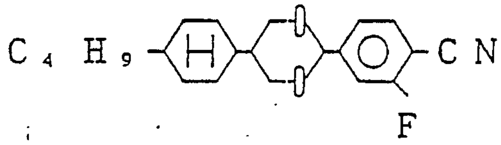 10 WT %

Liquid crystal composition [D-5]

〔M－5〕 90 WT %

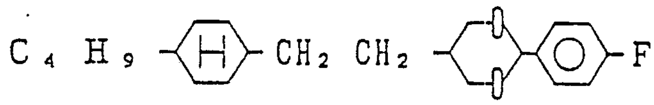 10 WT %

Liquid crystal composition [E-5]

〔M－5〕 90 WT %

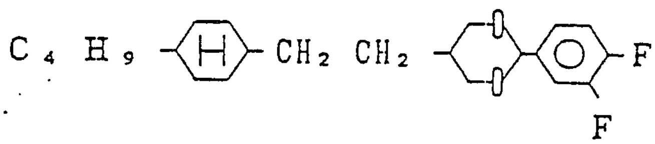 10 WT %

Liquid crystal composition [F-5]

〔M－5〕 90 WT %

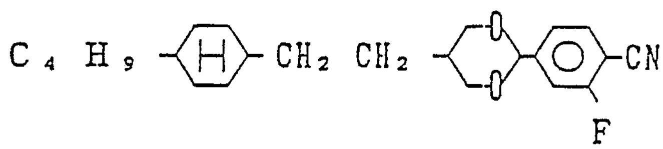 10 WT %

Liquid crystal composition [I-5]

〔M－5〕 90 WT %

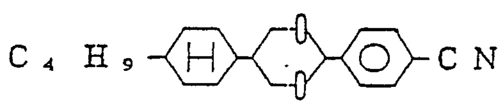 10 WT %

Liquid crystal composition [II-5]

[M − 5] 90 WT %

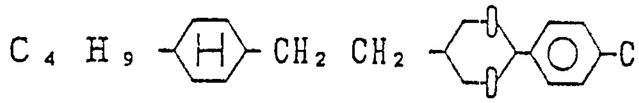 10 WT %

Liquid crystal composition [III-5]

[M − 5] 90 WT %

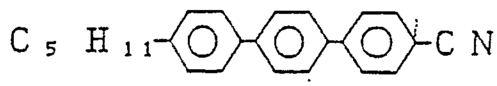 10 WT %

Liquid crystal composition [IV-5]

[M − 5] 90 WT %

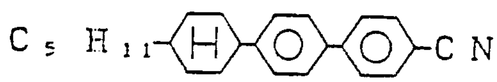 10 WT %

These liquid crystal compositions were tested for N-I point, Δn, $V_{th}$, α, γ, $\tau_{ON}$, $\tau_{OFF}$, ΔT and M. The results were as"shown collectively in Table 8.

Table 8

| Composition | N-I POINT (°C) | Δ n | $V_{th}$(V) | α | γ | $\tau_{ON}$(ms) | $\tau_{OFF}$ (ms) | ΔT(V) | M(%) |
|---|---|---|---|---|---|---|---|---|---|
| A-5 | 65.9 | 0.110 | 1.94 | 1.24 | 1.40 | 121 | 247 | -0.34 | 10.3 |
| B-5 | 64.2 | 0.108 | 1.89 | 1.23 | 1.39 | 123 | 240 | -0.38 | 9.4 |
| C-5 | 69.0 | 0.114 | 1.75 | 1.24 | 1.39 | 119 | 234 | -0.35 | 10.0 |
| D-5 | 64.8 | 0.108 | 1.94 | 1.24 | 1.41 | 104 | 210 | -0.33 | 10.7 |
| E-5 | 62.7 | 0.108 | 1.92 | 1.24 | 1.43 | 118 | 248 | -0.37 | 9.4 |
| F-5 | 67.5 | 0.114 | 1.81 | 1.26 | 1.44 | 123 | 256 | -0.36 | 8.4 |
| I-5 | 72.5 | 0.116 | 1.87 | 1.24 | 1.38 | 128 | 240 | -0.34 | 10.0 |
| II-5 | 70.6 | 0.116 | 1.90 | 1.25 | 1.40 | 125 | 253 | -0.33 | 10.0 |
| III-5 | 74.4 | 0.136 | 2.03 | 1.27 | 1.47 | 108 | 228 | -0.33 | 9.2 |
| IV-5 | 73.8 | 0.124 | 2.03 | 1.28 | 1.47 | 108 | 238 | -0.32 | 9.7 |

Example 17

A liquid crystal composition was prepared by mixing Nn type phenylcyclohexane carboxylic esters, Np type cyanophenyldioxanes, Np type cyanophenylcyclohexane carboxylic esters and the compound (1-c) of the present invention as shown below.

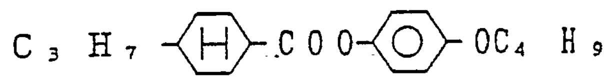 8.9 WT %

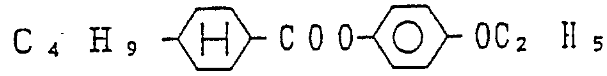 7.0 WT %

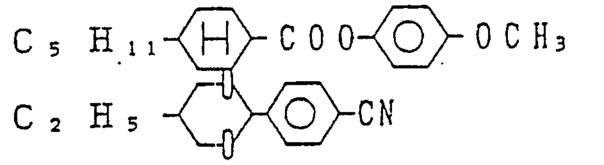 7.5 WT %

10.4 WT %

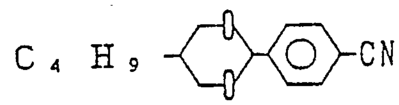 15.6 WT %

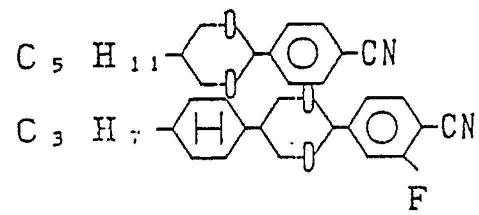 15.6 WT %

10.0 WT %

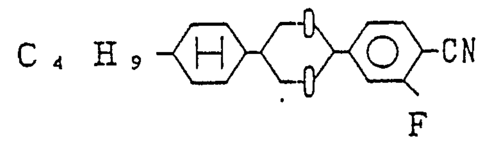 10.0 WT %

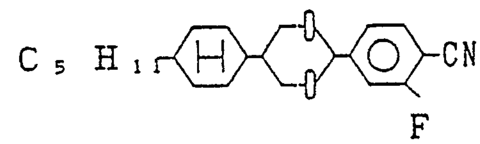 10.0 WT %

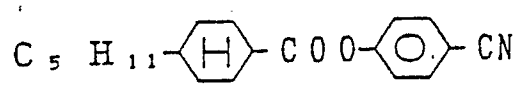 5.0 WT %

This liquid crystal composition showed a N-I point of 79.5° C and a Δn of 0.112. When it was sealed in a TN type cell 6.3 μm in thickness and tested at 20° C for $V\text{-}I_o$ characteristics, it showed a $V_{th}$ of 1.24 V, an α of 1.29, a γ of 1.45, a $\tau_{ON}$ of 77.3 ms, a $\tau_{OFF}$ of 162 ms, a ΔT of -0.377 V, and a M of 7.5%.

Example 18

A liquid crystal composition was prepared by mixing Nm type phenylcyclohexane carboxylic esters, Np type cyanophenyldioxanes, Np type cyanophenylcyclohexane carboxylic esters, Nn type phenylcyclohexylcyclohexane carboxylic esters and the compound (1-c) of the present invention as shown below.

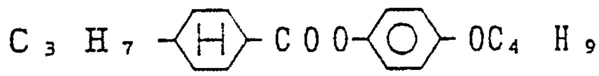 10.3 WT %

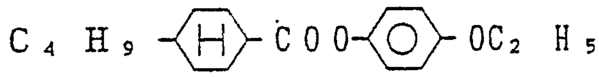 8.0 WT %

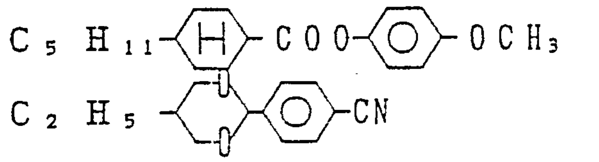 8.7 WT %

12.0 WT %

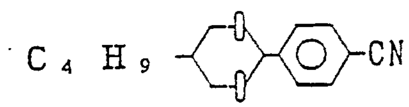 18.0 WT %

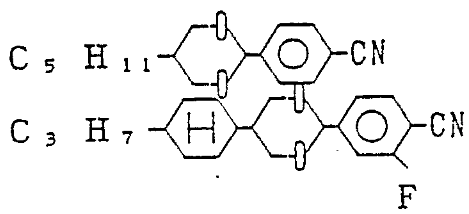 18.0 WT %

5.0 WT %

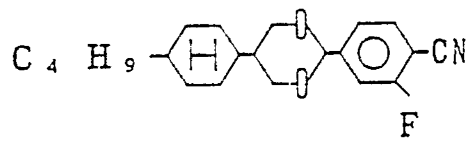 5.0 WT %

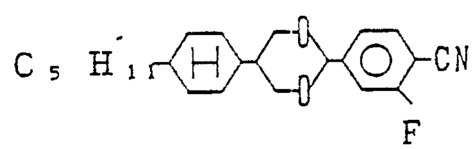 5.0 WT %

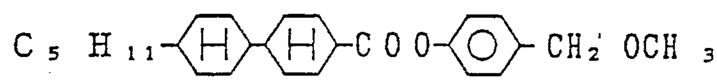 5.0 WT %

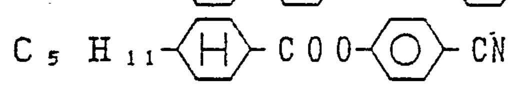 5.0 WT %

This liquid crystal composition showed a N-I point of 67.4° C and a $\Delta n$ of 0.105. When it was sealed in a TN type cell 6.3 $\mu$m in thickness and tested at 20° C for V-$I_0$ characteristics, it showed a $V_{th}$ of 1.20 V, an $\alpha$ of 1.26, a $\gamma$ of 1.45, a $\tau_{ON}$ of 66.6 ms, a $\tau_{OFF}$ of 135 ms, a $\Delta T$ of -0.46 V, and a M of 6.8%.

Example 19

A liquid crystal composition was prepared by mixing Nn type phenylcyclohexane carboxylic esters, Np type cyanophenyldioxanes, Np type cyanophenylcyclohexane carboxylic acids, and the compound (1-f) of the present invention as shown below.

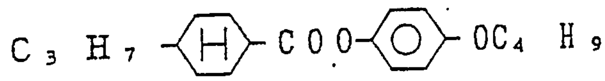 8.9 WT %

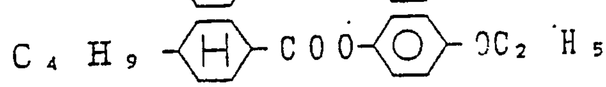 7.0 WT %

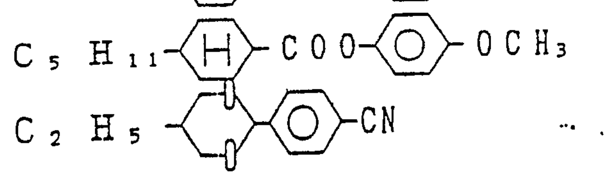 7.5 WT %

10.4 WT %

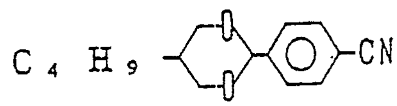 15.6 WT %

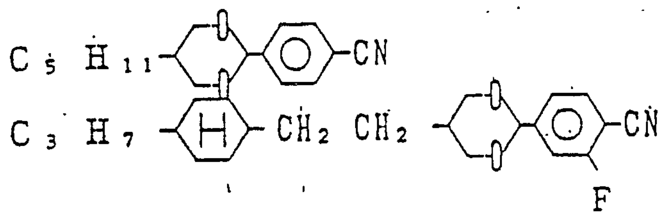 15.6 WT %

10.0 WT %

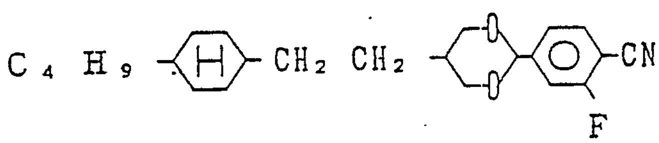 10.0 WT %

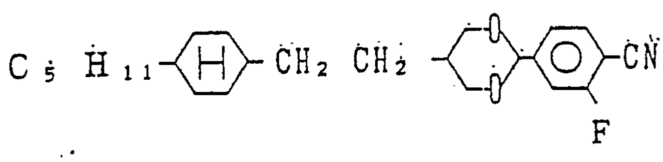 10.0 WT %

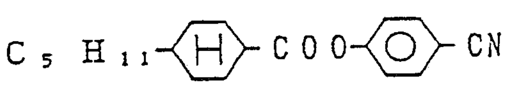 5.0 WT %

This liquid crystal composition showed a N-I point of 72.5° C and a Δn of 0.107. When it was sealed in a TN type cell 6.3 μm in thickness and tested at 20° C for V-$I_o$ characteristics, it showed a $V_{th}$ of 1.21 V, an α of 1.28, a γ of 1.45, a $\tau_{ON}$ of 80.6 ms, a $\tau_{OFF}$ of 162 ms, a ΔT of -0.383 V, and a M of 7.0%.

Example 20

A liquid crystal composition was prepared by mixing Nn type phenylcyclohexane carboxylic esters, Np type cyanophenyldioxanes, Nn type phenylcyclohexylcyclohexane carboxylic esters, Np type cyanophenylcyclohexane carboxylic esters, and the compound (1-f) of the present invention as shown below.

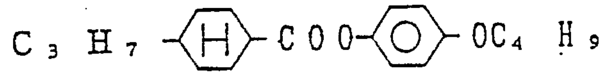 10.3 WT %

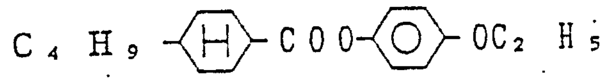 8.0 WT %

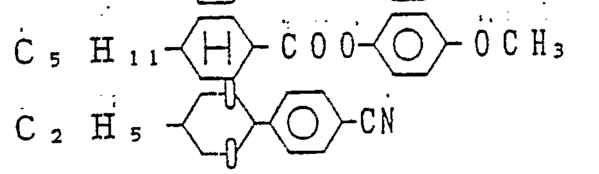 8.7 WT %

12.8 WT %

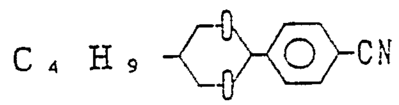 18.0 WT %

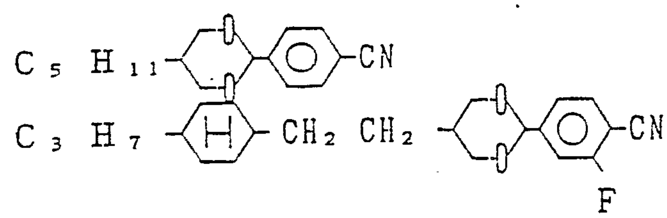 18.0 WT %

5.0 WT %

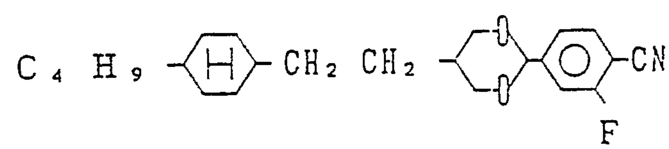 5.0 WT %

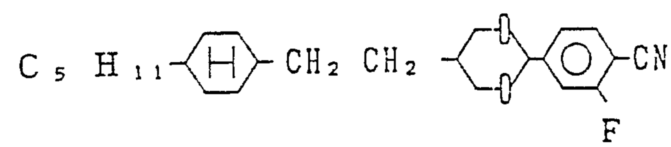 5.0 WT %

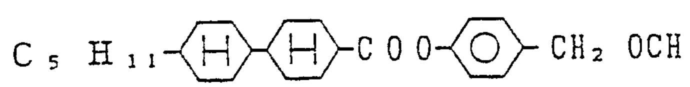 5.0 WT %

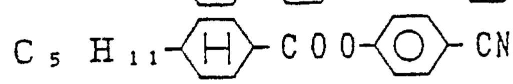 5.0 WT %

This liquid crystal composition showed a N-I point of 64.7°C and a Δn of 0.103. When it was sealed in a TN type cell 6.3 μm in thickness and tested at 20°C for $V\text{-}I_0$ characteristics, it showed a $V_{th}$ of 1.15 V, an α of 1.25, a γ of 1.45, a $\tau_{ON}$ of 71.1 ms, a $\tau_{OFF}$ of 139 ms, a ΔT of -0.467 V, and a M of 6.8%.

It is noted from the results of Examples 17 to 20 that the liquid crystal compositions incorporating 1,3-dioxane derivatives of the present invention possess small magnitudes of Δn and are capable of high time shared drive at a low voltage.

It has been demonstrated that the 1,3-dioxane derivatives of the present invention possess N-I points in the range of 80° to 180°C, that those of the derivatives involving substitution of F possess particularly small magnitudes of Δn, and that those of the derivatives having F substituted at the 3 position and CN at the 4 position possess very large magnitudes of Δε. It has been further confirmed that liquid crystal compositions obtained by mixing 1,3-dioxane derivatives according to the present invention with conventional liquid crystal compounds possess wide visual angles and permit high time shared drive at a low voltage.

The 1,3-dioxane derivatives according to the present invention, therefore, are highly useful as liquid crystal materials for TN type and STN type liquid crystal display devices.

## Claims

1. A 1,3-dioxane derivative characterised by the general formula:

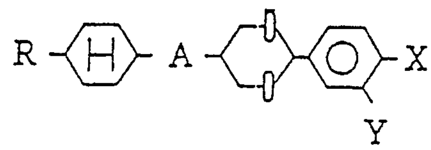

wherein, R is a straight chain alkyl group of 1 to 10 carbon atoms, A is a single bond or a $-CH_2-CH_2-$ group, X is F or CN, Y is either F when X is CN or H or F when X is F, and the cyclohexane ring and the 1,3-dioxane ring are each in the trans configuration.

2. A 1,3-dioxane derivative as claimed in claim 1 characterised by the general formula:

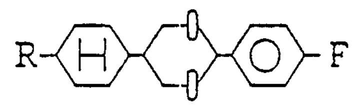

3. A 1,3-dioxane derivative as claimed in claim 1 characterised by the general formula:

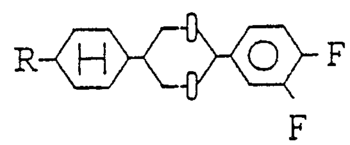

4. A 1,3-dioxane derivative as claimed in claim 1 characterised by the general formula:

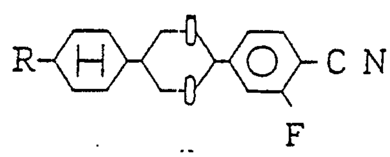

5. A 1,3-dioxane derivative as claimed in claim 1 characterised by the general formula:

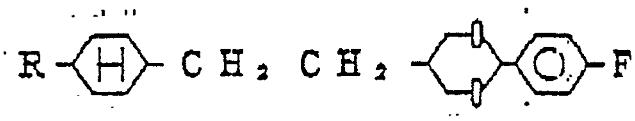

6. A 1,3-dioxane derivative as claimed in claim 1 characterised by the general formula:

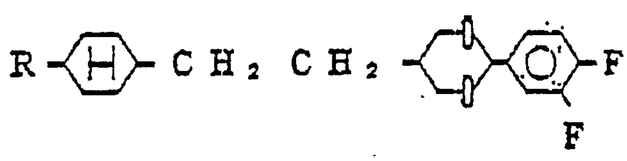

7. A 1,3-dioxane derivative as claimed in claim 1 characterised by the general formula:

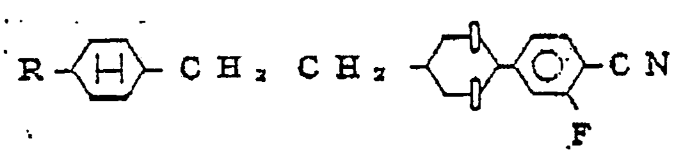

8. Trans-2-(4′-fluorophenyl)-5-(trans-4′-methylcyclohexyl)-1,3-dioxane, or
trans-2-(4′-fluorophenyl)-5-(trans-4′-ethylcyclo hexyl)-1,3-dioxane, or
trans-2-(4′-fluorophenyl)-5-(trans-4′-butylcyclohexyl)-1,3-dioxane, or

trans-2-(4′-fluorophenyl)-5-(trans-4′-propylcyclohexyl)-1,3-dioxane, or
trans-2-(4′-fluorophenyl)-5-(trans-4′-pentylcyclohexyl)-1,3-dioxane, or
trans-2-(4′-fluorophenyl)-5-(trans-4′-hexylcyclohexyl)-1,3-dioxane, or
trans-2-(4′-fluorophenyl)-5-(trans-4′-heptylcyclohexyl)-1,3-dioxane, or
trans-2-(4′-fluorophenyl)-5-(trans-4′-octylcyclohexyl)-1,3-dioxane, or
trans-2-(4′-fluorophenyl)-5-(trans-4′-nonylcyclohexyl)-1,3-dioxane, or
trans-2-(4′-fluorophenyl)-5-(trans-4′-decylcyclohexyl)-1,3-dioxane, or
trans-2-(3′,4′-difluorophenyl)-5-(trans-4′-methylcyclohexyl)-1,3-dioxane, or
trans-2-(3′,4′-difluorophenyl)-5-(trans-4′-ethylcyclohexyl)-1,3-dioxane, or
trans-2-(3′,4′-difluorophenyl)-5-(trans-4′-butylcyclohexyl)-1,3-dioxane, or
trans-2-(3′,4′-difluorophenyl)-5-(trans-4′-propylcyclohexyl)-1,3-dioxane, or
trans-2-(3′,4′-difluorophenyl)-5-(trans-4′-pentylcyclohexyl)-1,3-dioxane, or
trans-2-(3′,4′-difluorophenyl)-5-(trans-4′-hexylcyclohexyl)-1,3-dioxane, or
trans-2-(3′,4′-difluorophenyl)-5-(trans-4′-heptylcyclohexyl)-1,3-dioxane, or
trans-2-(3′,4′-difluorophenyl)-5-(trans-4′-octylcyclohexyl)-1,3-dioxane, or
trans-2-(3′,4′-difluorophenyl)-5-(trans-4′-nonylcyclohexyl)-1,3-dioxane, or
trans-2-(3′,4′-difluorophenyl)-5-(trans-4′-decylcyclohexyl)-1,3-dioxane, or
trans-2-(4′-cyano-3′-fluorophenyl-5-(trans-4′-methylcyclohexyl)-1,3-dioxane, or
trans-2-(4′-cyano-3′-fluorophenyl-5-(trans-4′-ethylcyclohexyl)-1,3-dioxane, or
trans-2-(4′-cyano-3′-fluorophenyl-5-(trans-4′-butylcyclohexyl)-1,3-dioxane, or
trans-2-(4′-cyano-3′-fluorophenyl-5-(trans-4′-propylcyclohexyl)-1,3-dioxane, or
trans-2-(4′-cyano-3′-fluorophenyl-5-(trans-4′-pentylcyclohexyl)-1,3-dioxane, or
trans-2-(4′-cyano-3′-fluorophenyl-5-(trans-4′-hexylcyclohexyl)-1,3-dioxane, or
trans-2-(4′-cyano-3′-fluorophenyl-5-(trans-4′-heptylcyclohexyl)-1,3-dioxane, or
trans-2-(4′-cyano-3′-fluorophenyl-5-(trans-4′-octylcyclohexyl)-1,3-dioxane, or
trans-2-(4′-cyano-3′-fluorophenyl-5-(trans-4′-nonylcyclohexyl)-1,3-dioxane, or
trans-2-(4′-cyano-3′-fluorophenyl-5-(trans-4′-decylcyclohexyl)-1,3-dioxane, or
trans-2-(4′-fluorophenyl)-5-[2′-(trans-4′-methylcyclohexyl)-ethyl]-1,3-dioxane, or
trans-2-(4′-fluorophenyl)-5-[2′-(trans-4′-ethylcyclohexyl)-ethyl]-1,3-dioxane, or
trans-2-(4′-fluorophenyl)-5-[2′-(trans-4′-butylcyclohexyl)-ethyl]-1,3-dioxane, or
trans-2-(4′-fluorophenyl)-5-[2′-(trans-4′-propylcyclohexyl)-ethyl]-1,3-dioxane, or
trans-2-(4′-fluorophenyl)-5-[2′-(trans-4′-pentylcyclohexyl)-ethyl]-1,3-dioxane, or
trans-2-(4′-fluorophenyl)-5-[2′-(trans-4′-hexylcyclohexyl)-ethyl]-1,3-dioxane, or
trans-2-(4′-fluorophenyl)-5-[2′-(trans-4′-heptylcyclohexyl)-ethyl]-1,3-dioxane, or
trans-2-(4′-fluorophenyl)-5-[2′-(trans-4′-octylcyclohexyl)-ethyl]-1,3-dioxane, or
trans-2-(4′-fluorophenyl)-5-[2′-(trans-4′-nonylcyclohexyl)-ethyl]-1,3-dioxane, or
trans-2-(4′-fluorophenyl)-5-[2′-(trans-4′-decylcyclohexyl)-ethyl]-1,3-dioxane, or
trans-2-(3′,4′-difluorophenyl)-5-[2′-(trans-4″-methylcyclohexyl)-ethyl]-1,3-dioxane, or
trans-2-(3′,4′-difluorophenyl)-5-[2′-(trans-4″-ethylcyclohexyl)-ethyl]-1,3-dioxane, or
trans-2-(3′,4′-difluorophenyl)-5-[2′-(trans-4″-butylcyclohexyl)-ethyl]-1,3-dioxane, or
trans-2-(3′,4′-difluorophenyl)-5-[2′-(trans-4″-propylcyclohexyl)-ethyl]-1,3-dioxane, or
trans-2-(3′,4′-difluorophenyl)-5-[2′-(trans-4″-pentylcyclohexyl)-ethyl]-1,3-dioxane, or
trans-2-(3′,4′-difluorophenyl)-5-[2′-(trans-4″-hexylcyclohexyl)-ethyl]-1,3-dioxane, or
trans-2-(3′,4′-difluorophenyl)-5-[2′-(trans-4″-heptylcyclohexyl)-ethyl]-1,3-dioxane, or
trans-2-(3′,4′-difluorophenyl)-5-[2′-(trans-4″-octylcyclohexyl)-ethyl]-1,3-dioxane, or
trans-2-(3′,4′-difluorophenyl)-5-[2′-(trans-4″-nonylcyclohexyl)-ethyl]-1,3-dioxane, or
trans-2-(3′,4′-difluorophenyl)-5-[2′-(trans-4″-decylcyclohexyl)-ethyl]-1,3-dioxane, or
trans-2-(4′-cyano-3′-fluorophenyl)-5-[2′-(trans-4″-methylcyclohexyl)-ethyl]-1,3-dioxane, or
trans-2-(4′-cyano-3′-fluorophenyl)-5-[2′-(trans-4″-ethylcyclohexyl)-ethyl]-1,3-dioxane, or
trans-2-(4′-cyano-3′-fluorophenyl)-5-[2′-(trans-4″-butylcyclohexyl)-ethyl]-1,3-dioxane, or
trans-2-(4′-cyano-3′-fluorophenyl)-5-[2′-(trans-4″-propylcyclohexyl)-ethyl]-1,3-dioxane, or
trans-2-(4′-cyano-3′-fluorophenyl)-5-[2′-(trans-4″-pentylcyclohexyl)-ethyl]-1,3-dioxane, or
trans-2-(4′-cyano-3′-fluorophenyl)-5-[2′-(trans-4″-hexylcyclohexyl)-ethyl]-1,3-dioxane, or
trans-2-(4′-cyano-3′-fluorophenyl)-5-[2′-(trans-4″-heptylcyclohexyl)-ethyl]-1,3-dioxane, or
trans-2-(4′-cyano-3′-fluorophenyl)-5-[2′-(trans-4″-octylcyclohexyl)-ethyl]-1,3-dioxane, or
trans-2-(4′-cyano-3′-fluorophenyl)-5-[2′-(trans-4″-nonylcyclohexyl)-ethyl]-1,3-dioxane, or
trans-2-(4′-cyano-3′-fluorophenyl)-5-[2′-(trans-4″-decylcyclohexyl)-ethyl]-1,3-dioxane.

9. A liquid crystal composition characterised by including at least one 1,3-dioxane derivative as claimed

in any preceding claim.

10. A liquid crystal composition characterised by including at least one 1,3-dioxane derivative as claimed in any of claims 1 to 8 in an amount of 3 to 30% by weight.

European Patent Office

# EUROPEAN SEARCH REPORT

Application number

EP 90305531.7

| Category | DOCUMENTS CONSIDERED TO BE RELEVANT<br>Citation of document with indication, where appropriate, of relevant passages | Relevant to claim | CLASSIFICATION OF THE APPLICATION (Int Cl 5) |
|---|---|---|---|
| A | DE - A1 - 3 227 916 (VEB WERK FÜR FERNSEHELEKTRONIK) * Claims * -- | 1,9,10 | C 07 D 319/06<br>C 09 K 19/30 |
| A | DE - A1 - 3 447 359 (MERCK PATENT GMBH) * Claims * -- | 1,9,10 | |
| A | GB - A - 2 138 838 (VEB WERK FÜR FERNSEHELEKTRONIK) * Abstract; pages 9,10, compounds 7/2,18/13 * ---- | 1,9,10 | |
| | | | TECHNICAL FIELDS SEARCHED (Int Cl 5) |
| | | | C 07 D 319/00<br>C 09 K 19/00 |

The present search report has been drawn up for all claims

| Place of search | Date of completion of the search | Examiner |
|---|---|---|
| VIENNA | 20-07-1990 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document